(19) Europäisches Patentamt
European Patent Office
Office européen des brevets

(11) Numéro de publication: **0 197 230 B1**

# (12) FASCICULE DE BREVET EUROPEEN

(45) Date de publication du fascicule du brevet: **31.01.90**

(21) Numéro de dépôt: **85402558.2**

(22) Date de dépôt: **19.12.85**

(51) Int. Cl.⁴: **C07D 487/04**, A61K 31/505 // (C07D487/04, 239:00, 235:00)

(54) **Imidazo /1,2-a/ pyrimidines et leurs sels avec les acides, procédé et intermédiaires de préparation, application comme médicaments et compositions les renfermant.**

(30) Priorité: **19.12.84 GB 8432073**

(43) Date de publication de la demande: **15.10.86 Bulletin 86/42**

(45) Mention de la délivrance du brevet: **31.01.90 Bulletin 90/5**

(84) Etats contractants désignés: **AT BE CH DE FR IT LI LU NL SE**

(56) Documents cités:
**EP-A- 0 054 507**
**EP-A- 0 104 104**

(73) Titulaire: **ROUSSEL-UCLAF, 35, boulevard des Invalides, F-75007 Paris(FR)**

(72) Inventeur: **Gillespie, Roger John, 22 Wentworth Park Freshbrook, Swindon SN5 8QX Wiltshire(GB)**
Inventeur: **Tully, Wilfred Roger, 5 Saint Peter's Road, Cirencester Gloucestershire(GB)**

(74) Mandataire: **Vieillefosse, Jean-Claude et al, Département des Brevets ROUSSEL UCLAF B.P no 9, F-93230 Romainville(FR)**

EP 0 197 230 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne des imidazo[1,2-a]pyrimidines et leurs sels, leur préparation, leur application à titre de médicament, les compositions les renfermant et les nouveaux intermédiaires obtenus.

La demande de brevet européen EP-A 0 104 104 décrit des imidazo[1,2-a]pyrimidines substituées en position 2 par un groupement –CO–R dans lequel R peut notamment représenter un radical aryle, thiényle ou pyridyle. Ces produits sont doués de propriétés anxiolytiques. La demande de brevet européen EP-A 0 054 507 décrit des béta carbolines substituées en 3 présentant des propriétés anxiolytiques.

L'invention a pour objet des imidazo[1,2-a]pyrimidines, ainsi que leurs sels d'addition avec les acides minéraux ou organiques, caractérisées en ce qu'elles répondent à la formule générale (I):

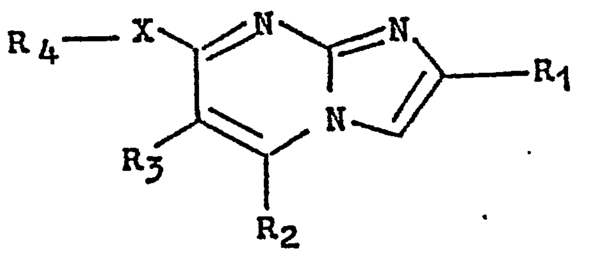

(I)

dans laquelle:

– $R_1$ représente un groupement 1,2,4-oxadiazol-5-yl ou 1,3,4-thiadiazol-2-yl éventuellement substitué par un radical alkyl renfermant de 1 à 3 atomes de carbone ou un radical alkényl renfermant de 2 à 5 atomes de carbone ou un groupement 1,2,4-oxadiazol-3-yl ou 1,3,4-oxadiazol-2-yl éventuellement substitué par un radical alkyl renfermant de 1 à 3 atomes de carbone lui-même éventuellement substitué par un ou plusieurs atomes de fluor, ou par un radical alkényle renfermant de 2 à 5 atomes de carbone,

– $R_2$, $R_3$, identiques ou différents, représentent un atome d'hydrogène, un radical alkyl renfermant de 1 à 3 atomes de carbone, un radical alkényl renfermant de 2 à 5 atomes de carbone, ou $R_2$ et $R_3$ forment ensemble un radical alkylène renfermant de 3 à 5 atomes de carbone,

– X représente un atome d'oxygène ou de soufre,

– $R_4$ représente un radical alkyl renfermant de 1 à 3 atomes de carbone.

Dans la formule (I) et dans ce qui suit:

– le terme radical alkyle renfermant 1 à 3 atomes de carbone désigne, par exemple, un radical méthyl, éthyl ou propyl,

– le terme radical alkyl substitué par un ou plusieurs atomes de fluor désigne notamment le radical trifluorométhyl,

– le terme radical alkényl renfermant de 2 à 5 atomes de carbone désigne, par exemple, un radical vinyl, allyl ou butényl,

– le terme radical alcoylène renfermant de 3 à 5 atomes de carbone désigne, par exemple, un radical propylène, butylène ou pentaméthylène.

Les produits de l'invention peuvent être sous forme de solvates, notamment d'hydrates.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzènesulfonique.

Parmi les produits, objet de l'invention, on peut citer notamment les dérivés répondant à la formule (I) ci-dessus, ainsi que leurs sels, dans laquelle $R_1$ représente un radical 5-méthyl, 1,3,4-oxadiazol-5-yl, 5-propyl 1,3,4-oxadiazol-2-yl, 5-trifluorométhyl 1,2,4-oxadiazol-3-yl, 5-trifluorométhyl 1,3,4-oxadiazol-2-yl ou 5-méthyl 1,3,4-thiadiazol-2-yl.

Parmi ces derniers, on peut citer les dérivés répondant à la formule (I) ci-dessus, ainsi que leurs sels, dans laquelle $R_1$ représente un radical 3-méthyl 1,2,4-oxadiazol-5-yl, un radical 5-méthyl 1,2,4-oxadiazol-3-yl, un radical 5-propyl 1,3,4-oxadiazol-2-yl, un radical 5-trifluorométhyl 1,2,4-oxadiazol-3-yl, un radical 5-méthyl 1,3,4-oxadiazol-2-yl ou un radical 5-méthyl 1,3,4-thiadiazol-2-yl.

Parmi les produits, objet de l'invention, on retient tout particulièrement les produits répondant à la formule (I) ci-dessus, ainsi que leurs sels, dans laquelle $R_2$ et $R_3$ représentent un radical alkyl renfermant de 1 à 3 atomes de carbone et X représente un atome d'oxygène et notamment la 6-éthyl 7-méthoxy 5-méthyl 2-(3-méthyl 1,2,4-oxadiazol-5-yl) imidazo[1,2-a]pyrimidine, la 6-éthyl 7-méthoxy 5-méthyl 2-(5-méthyl 1,2,4-oxadiazol-3-yl) imidazo[1,2-a]pyrimidine, la 6-éthyl 7 méthoxy 5-méthyl 2-(5-trifluorométhyl 1,2,'-oxadiazol-3-yl) imidazo[1,2-a]pyrimidine, la 6-éthyl 7-méthoxy 5-méthyl 2-(5-méthyl 1,3,4-oxadiazol-2-yl)

imidazo[1,2-a]pyrimidine et la 7-méthoxy 5-méthyl 2-(5-méthyl 1,2,4-oxadiazol-3-yl) 6-propylimidazo[1,2-a]pyrimidine.

Parmi les produits, objet de l'invention, on peut citer aussi tout particulièrement la 6-éthyl 5-méthyl 2-(5-méthyl 1,3,4-oxadiazol-2-yl) 7-méthylthioimidazo[1,2-a]pyrimidine, la 6-éthyl 5-méthyl 2-(5-méthyl 1,2,4-oxadiazol-3-yl) 7-méthylthioimidazo[1,2-a]pyrimidine, la 6,7,8,9-tétrahydro 5-méthoxy 2-(5-méthyl 1,2,4-oxadiazol-3-yl) imidazo[1,2-a]quinazoline et leurs sels.

L'invention a également pour objet un procédé de préparation des imidazo[1,2-a]pyrimidines telles que définies par la formule (I) ci-dessus, dans laquelle $R_1$ représente un radical 1,2,4-oxadiazol-5-yl ainsi que de leurs sels, caractérisé en ce que l'on fait réagir un produit de formule (II):

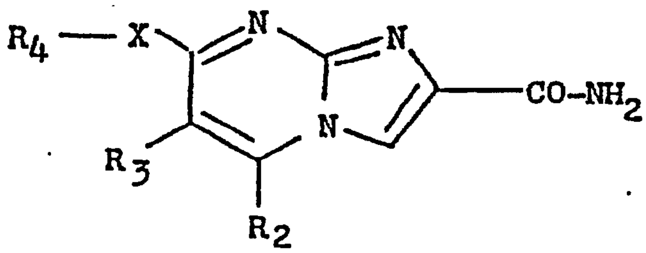

(II)

dans laquelle $R_2$, $R_3$, $R_4$ et X ont la signification déjà indiquée, avec un produit de formule (III):

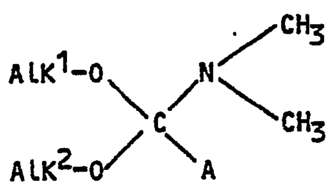

(III)

dans laquelle $ALK^1$ et $ALK^2$ représentent chacun un radical alkyl renfermant de 1 à 3 atomes de carbone et A représente un atome d'hydrogène, un radical alkyl renfermant de 1 à 3 atomes de carbone ou un radical alkényl renfermant de 2 à 5 atomes de carbone, pour obtenir un produit de formule (IV):

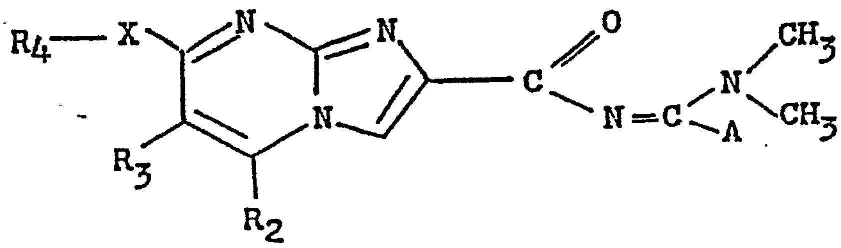

(IV)

dans laquelle $R_2$, $R_3$, $R_4$, X et A ont la signification déjà indiquée, puis fait réagir ce dernier avec le chlorhydrate d'hydroxylamine, pour obtenir le produit de formule ($I_A$):

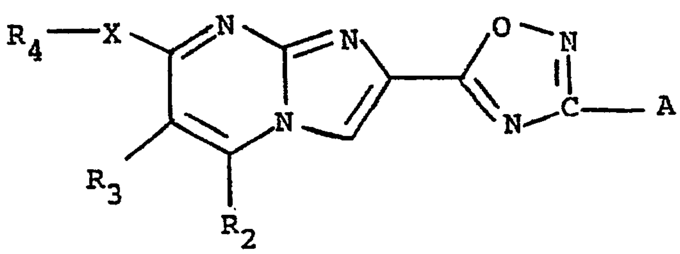

($I_A$)

recherché, dans laquelle $R_2$, $R_3$, $R_4$, X et A ont la signification déjà indiquée, puis le cas échéant, salifie le produit de formule (I) ainsi obtenu.

Dans des conditions préférentielles de mise en œuvre de l'invention, le procédé de préparation ci-dessus décrit est caractérisé en ce que:

a) la réaction du produit de formule (II) avec le produit de formule (III) est effectuée par chauffage du milieu réactionnel;

b) la réaction du produit de formule (IV) avec le chlorhydrate d'hydroxylamine est effectuée en présence d'une base telle que l'hydroxyde de sodium au sein d'un solvant organique tel que le dioxanne en présence d'un cosolvant tel que l'acide acétique; le produit de formule ($I_A$) peut être préparé sans isoler le produit intermédiaire.

L'invention a également pour objet un procédé de préparation des imidazo[1,2-a]pyrimidines telles que définies par la formule (I) ci-dessus, dans laquelle $R_1$ représente un radical 1,2,4-oxadiazol-3-yl, ainsi que de leurs sels, caractérisé en ce que l'on fait réagir un produit de formule (V):

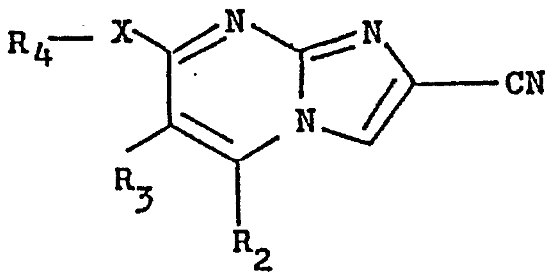

(V)

dans laquelle $R_2$, $R_3$, $R_4$ et X ont la signification déjà indiquée, avec le chlorhydrate d'hydroxylamine, pour obtenir un produit de formule (VI):

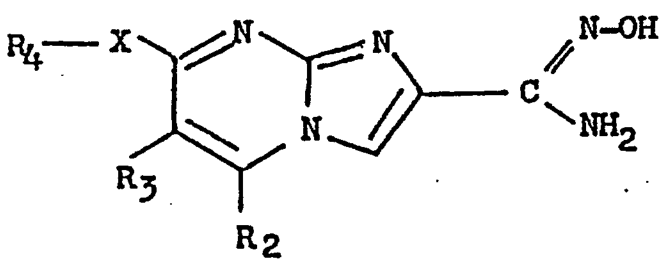

(VI)

dans laquelle $R_2$, $R_3$, $R_4$ et X ont la signification déjà indiquée, puis fait réagir ce dernier avec un dérivé fonctionnel d'un acide de formule (IX) :

D-COOH (IX)

dans laquelle D représente un atome d'hydrogène, un radical alkyl renfermant de 1 à 3 atomes de carbone éventuellement substitué par un ou plusieurs atomes de fluor ou un radical alkényl renfermant de 2 à 5 atomes de carbone pour obtenir un produit de formule ($I_B$) :

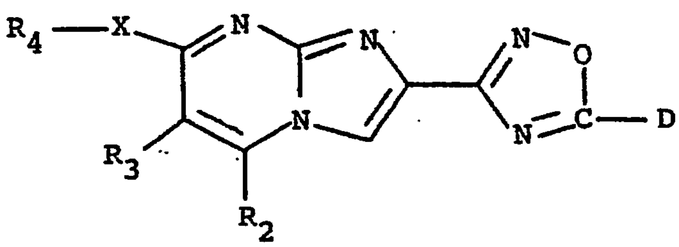

($I_B$)

dans laquelle $R_2$, $R_3$, $R_4$, X et D ont la signification déjà indiquée, puis le cas échéant, salifie le produit de formule (I) ainsi obtenu.

Dans des conditions préférentielles de mise en oeuvre de l'invention, le procédé de préparation cidessus décrit est caractérisé en ce que :

a) la réaction du produit de formule (V) avec le chlorhydrate d'hydroxylamine est effectuée par chauffage au reflux du mélange réactionnel au sein d'un solvant organique tel que l'éthanol, en présence d'une base telle que l'hydroxyde de potassium ;

b) le dérivé fonctionnel de l'acide de formule (IX)

D-COOH (IX)

est un anhydride d'acide ou un amide acétal de formule (III') :

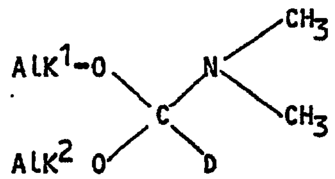

(III')

dans laquelle ALK[1] et ALK[2] ont les significations précédentes;

c) la réaction du produit de formule (VI) avec le dérivé fonctionnel de l'acide D–COOH est effectuée par chauffage du milieu réactionnel;

d) la réaction du produit de formule (VI) avec le dérivé fonctionnel de l'acide D–COOH est effectuée en présence d'une base telle que le triéthylamine au sein d'un solvant organique au reflus tel que le dichlorométhane, si le dérivé réactif est un agent déshydratant fort, par exemple l'anhydride trifluoroacétique.

L'invention a également pour objet un procédé de préparation des imidazo[1,2-a]pyrimidines telles que définies par la formule (I) ci-dessus, dans laquelle $R_1$ représente un radical 1,3,4-oxadiazol-2-yl, ainsi que de leurs sels, caractérisé en ce que l'on fait réagir un produit de formule (VII):

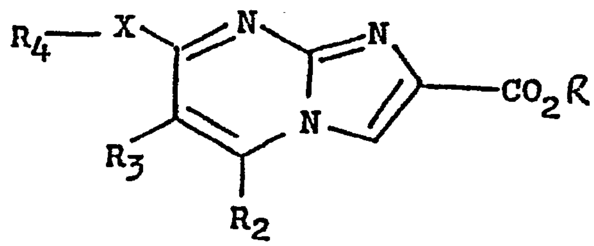

(VII)

dans laquelle $R_2$, $R_3$, $R_4$ et X ont la signification déjà indiquée et R représente le reste d'un groupement ester avec de l'hydrate d'hydrazine pour obtenir un produit de formule (VIII):

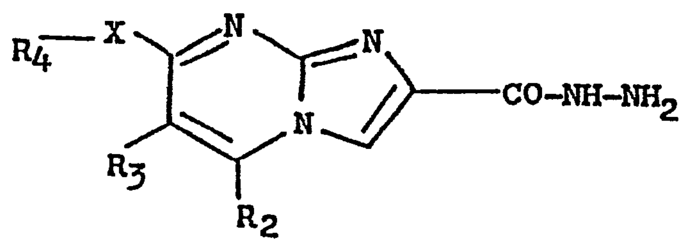

(VIII)

dans laquelle $R_2$, $R_3$, $R_4$ et X ont la signification déjà indiquée, puis fait réagir ce dernier avec soit un anhydride ou un chlorure d'acide de formule (IX):

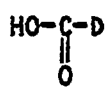

(IX)

dans laquelle D a la signification déjà indiquée, pour obtenir un produit de formule (X) :

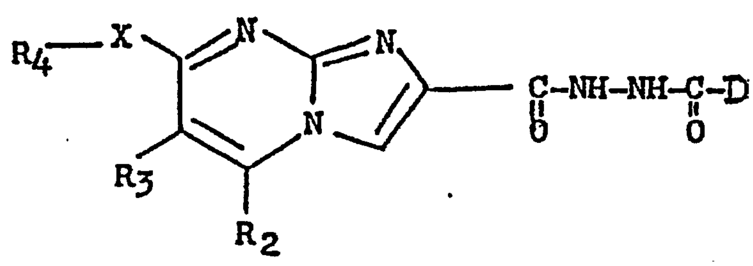

(X)

soit avec un orthoester de formule

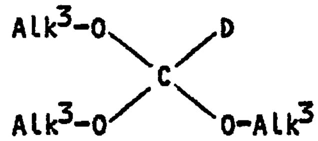

dans lequel $Alk^3$ représente un radical alkyle renfermant de 1 à 3 atomes de carbone pour obtenir un produit de formule (XI) :

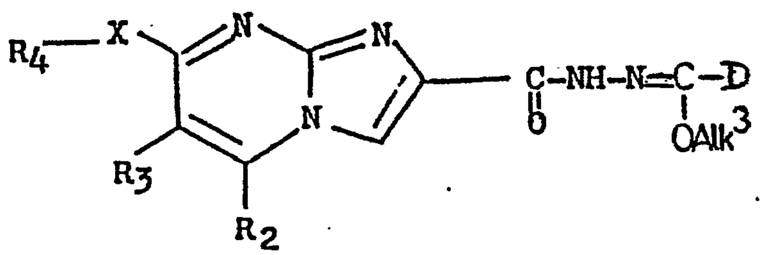

(XI)

dans laquelle $R_2$, $R_3$, $R_4$, X et D ont la signification déjà indiquée et $Alk^3$ a la signification précédente puis cyclise par chauffage le produit de formule (X) ou (XI), pour obtenir un produit de formule ($I_C$) :

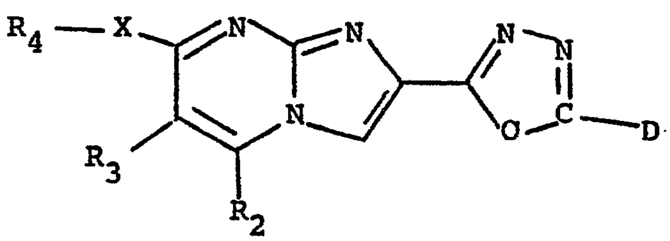

($I_C$)

dans laquelle $R_2$, $R_3$, $R_4$, X et D ont la signification déjà indiquée, puis le cas échéant, salifie le produit de formule (I) ainsi obtenu.

Dans des conditions préférentielles de mise en œuvre de l'invention, le procédé de préparation ci-dessus décrit est caractérisé en ce que:

a) le reste du groupement ester que représente R est un radical alkyle renfermant de 1 à 3 atomes de carbone, notamment le radical éthyle;

b) la réaction du produit de formule (VII) avec l'hydrate d'hydrazine est effectuée par chauffage au reflux du mélange réactionnel au sein d'un solvant organique tel que l'éthanol;

c) quand le dérivé réactif que l'on fait agir sur le produit de formule (VIII) est agent déshydratant fort tel que l'anhydride trifluoroacétique, le produit de formule (X) ou (XI) est préparé in situ dans le milieu réactionnel sans être isolé et la cyclisation est effectuée en présence d'une base telle que la triéthylamine au sein d'un solvant organique tel que le dichlorométhane à une température comprise entre 25° et 45°C;

d) quand le dérivé réactif que l'on fait réagir sur le produit de formule (VIII) n'est pas un agent déshydratant fort, le produit de formule (X) ou (XI) est isolé et la réaction de cyclisation est effectuée par chauffage du milieu réactionnel à 120°C ou plus, en présence de chlorure de phosphoryl ou de préférence en présence d'un acide fort tel que l'acide polyphosphorique.

L'invention a encore pour objet un procédé de préparation des imidazo[1,2-a]pyrimidines telles que définies par la formule (I) ci-dessus, dans laquelle $R_1$ représente un radical 1,3,4-thiadiazol-2-yl, ainsi que de leurs sels, caractérisé en ce que l'on fait réagir un produit de formule (VIII):

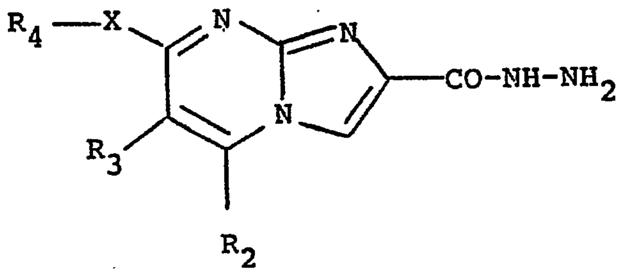

(VIII)

dans laquelle $R_2$, $R_3$, $R_4$ et X ont la signification déjà indiquée avec le dérivé fonctionnel d'un acide de formule (IX'):

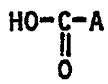

(IX')

dans laquelle A a la signification déjà indiquée, pour obtenir un produit de formule (XII) :

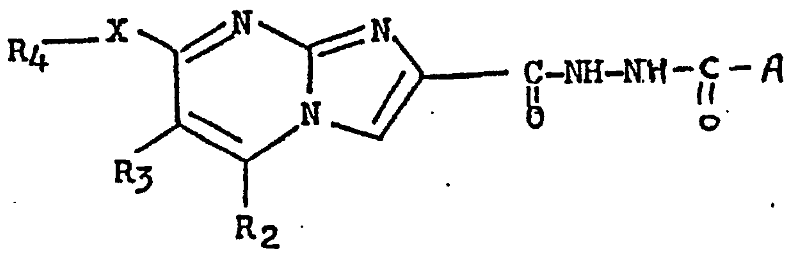

(XII)

dans laquelle $R_2$, $R_3$, $R_4$, X et A ont la signification déjà indiquée, puis fait réagir ce dernier avec le réactif de Lawesson au sein d'un solvant organique pour obtenir un produit de formule ($I_D$) :

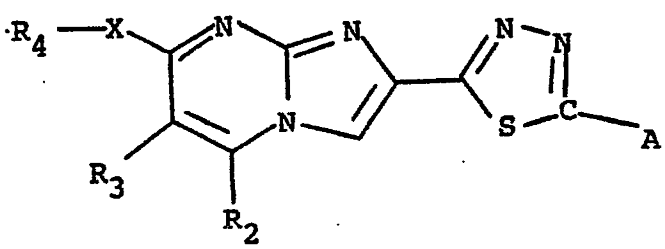

($I_D$)

dans laquelle $R_2$, $R_3$, $R_4$, X et A ont la signification déjà indiquée, puis le cas échéant, salifie le produit de formule (I) ainsi obtenu.

Dans des conditions préférentielles de mise en oeuvre de l'invention, le procédé de préparation ci-dessus décrit est caractérisé en ce que :

- la réaction avec le réactif de Lawesson ou (2,4-bis 4-méthoxyphényl) 1,3-dithia 2,4-diphosphétane 2,4-disulphide) de formule :

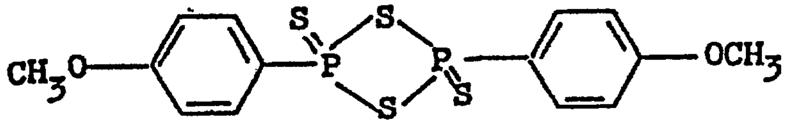

est effectuée au sein d'un solvant organique tel que le toluène, au reflux du mélange réactionnel.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques; ils sont doués notamment de remarquables propriétés anxiolytiques. Certains produits possèdent en outre des propriétés sédatives et hypnotiques.

Ces propriétés sont illustrées plus loin dans la partie expérimentale.

Ces propriétés justifient l'utilisation des imidazo[1,2-a]pyrimidines de formule (I), ainsi que de leurs sels, pharmaceutiquement acceptables, à titre de médicaments.

La présente invention a ainsi également pour objet l'application à titre de médicaments des imidazo[1,2-a]pyrimidines telles que définies par la formule générale (I) ainsi que de leurs sels d'addition avec les acides pharmaceutiquement acceptables.

Parmi ces médicaments, objet de l'invention, on retient notamment les médicaments, caractérisés en ce qu'ils sont constitués par les imidazo[1,2-a]pyrimidines répondant à la formule (I) dans laquelle $R_1$ représente un radical méthyloxadiazolyl, propyloxadiazolyl, 5-trifluorométhyl 1,2,4-oxadiazol-3-yl, 5-trifluorométhyl 1,3,4-oxadiazol-2-yl ou méthylthiadiazolyl, ainsi que leurs sels d'addition avec les acides pharmaceutiquement acceptables.

Parmi les médicaments de l'invention, on retient tout particulièrement ceux répondant à la formule (I) dans laquelle $R_1$ représente un radical 3-méthyl 1,2,4-oxadiazol-5-yl, un radical 5-méthyl 1,2,4-oxadiazol-3-yl, un radical 5-propyl 1,3,4-oxadiazol-2-yl, un radical 5-trifluorométhyl 1,2,4-oxadiazol-3-yl, un radical 5-méthyl 1,3,4-oxadiazol-2-yl ou un radical 5-méthyl 1,3,4-thiadiazol-2-yl, ainsi que leurs sels d'addition avec les acides pharmaceutiquement acceptables.

Parmi les médicaments préférés de l'invention, on retient tout particulièrement les produits de formule (I) dans laquelle $R_2$ et $R_3$ représentent un radical alkyl refermant de 1 à 3 atomes de carbon et X représente un atome d'oxygène, ainsi que leurs sels d'addition avec les acides pharmaceutiquement acceptables, et notamment:

– la 6-éthyl 7-méthoxy 5-méthyl 2-(3-méthyl 1,2,4-oxadiazol-5-yl) imidazo[1,2-a]pyrimidine, la 6-éthyl 7-méthoxy 5-méthyl 2-(5-méthyl 1,2,4-oxadiazol-3-yl) imidazo[1,2-a]pyrimidine, la 6-éthyl 7-méthoxy 5-méthyl 2-(5-trifluorométhyl 1,2,4-oxadiazol-3-yl) imidazo[1,2-a]pyrimidine, la 6-éthyl 7-méthoxy 5-méthyl 2-(5-méthyl 1,3,4-oxadiazol-2-yl) imidazo[1,2-a]pyrimidine et la 7-méthoxy 5-méthyl 2-(5-méthyl 1,2,4-oxadiazol-3-yl) 6-propylimidazo[1,2-a]pyrimidine.

Parmi les médicaments préférés de l'invention, on peut encore citer la 6-éthyl 5-méthyl 2-(5-méthyl 1,3,4-oxadiazol-2-yl) 7-méthylthioimidazo /1,2-a/ pyrimidine, la 6-éthyl 5-méthyl 2-(5-méthyl 1,2,4-oxadiazol-3-yl 7-méthylthioimidazo /1,2-a/ pyrimidine, la 6,7,8,9-tétrahydro 5-méthoxy 2-(5-méthyl 1,2,4-oxadiazol-3-yl imidazo /1,2-a/ quinazoline et leurs sels d'addition avec les acides pharmaceutiquement acceptables.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains produits de formule (II) ont été décrits dans Il Farmaco Ed. Sci. 1980, 35, 654. Lorsqu'ils ne sont pas connus, les produits de formule (II) peuvent être préparés par un procédé analogue, au départ du produit de formule (V) correspondant.

Un exemple d'une telle préparation est donné plus loin dans la partie expérimentale.

Les produits de formule (V) sont, par contre, nouveaux et peuvent être préparés par réaction d'un produit de formule (XIV) :

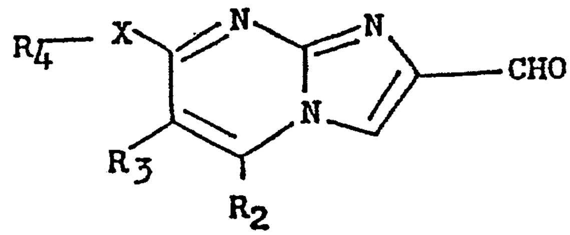

(XIV)

dans laquelle $R_2$, $R_3$, $R_4$ et X ont la signification déjà indiquée avec de l'o-sulfonate d'hydroxylamine dans l'eau suivie d'une alcalinisation par de l'hydroxyde de sodium.

Un exemple d'une telle préparation figure plus loin dans la partie expérimentale.

Les produits de formule (VII) et (XIV) ont quant à eux été décrits dans la littérature. Ils peuvent, par exemple, être préparés comme indiqué dans la demande de brevet britannique publiée sous le n° 2 128 989 A.

Il va être donné maintenant, à titre non limitatif, des exemples de mise en oeuvre de l'invention.

Exemple 1 :

(i) 6-éthyl 7-méthoxy 5-méthylimidazo /1,2-a/ pyrimidine 2-carboxamide.

Stade A : 6-éthyl 7-méthoxy 5-méthylimidazo /1,2-a/ pyrimidine 2-carbonitrile.

Dans une suspension comprenant 19,84 g de 6-éthyl 7-méthoxy 5-méthylimidazo /1,2-a/ pyrimidine 2-carboxaldéhyde dans 300 cm3 d'eau, on ajoute sous agitation à température ambiante, 13,3 g d'acide hydroxylamine O-sulfonique en solution dans 250 cm3 d'eau. Après 40 minutes d'agitation, on ajoute 8 g de soude, agite une heure, filtre le précipité, le lave à l'eau, le sèche, le recristallise dans l'acétate d'éthyle et obtient 16,18 g de produit attendu. F = 166-168°C.

Analyse : $C_{11}H_{12}N_4O$

Calculé : C% 61,10 H% 5,60 N% 25,91

Trouvé : 61,11 5,65 25,94.

Stade B : 6-éthyl 7-méthoxy 5-méthylimidazo /1,2-a/ pyrimidine 2-carboxamide.

On chauffe au reflux pendant 4 heures et demie une suspension comprenant 10 g de produit obtenu au stade A et 20 g de résine Amberlite IRA-400 sous forme de base dans 200 cm3 d'eau. On refroidit le mélange, ajoute du chloroforme pour dissoudre le précipité et élimine par filtration la résine. On sépare les phases par décantation, extrait la phase aqueuse au chloroforme, sèche et concentre à sec. Après recristallisation dans le méthanol, on obtient 10,5 g de produit attendu. F = 256-259°C.

Analyse : $C_{11}H_{14}N_4O_2$

Calculé : C% 56,40 H% 6,02 N% 23,92

Trouvé : 56,27 6,01 23,79.

(ii) 6-éthyl 7-méthoxy 5-méthyl 2-(3-méthyl 1,2,4-oxadiazol-5-yl) imidazo /1,2-a/ pyrimidine.

On agite 1 heure à 110°C, 5,4 g de 6-éthyl 7-méthoxy 5-méthylimidazo /1,2-a/ pyrimidine 2-carboxamide préparé ci-dessus et 11 g de diméthyl acétal de diméthylacétamide. On évapore à sec le mélange, reprend le résidu dans 2,24 g de chlorhydrate d'hydroxylamine, 40 cm3 de dioxanne, 40 cm3 d'acide acétique et 16,7 cm3 d'une solution aqueuse d'hydroxyde de sodium (2N). On chauffe la solution à 90°C pendant 1 heure et 45 minutes, on ajoute 60 cm3 d'eau, glace, sépare le produit cristallisé et isole 3,97 g de produit attendu. F = 204-205°C.

Exemple 2 : 6-éthyl 7-méthoxy 5-méthyl 2-(5-méthyl 1,2,4-oxadiazol-3-yl) imidazo /1,2-a/ pyrimidine.

Stade A : 6-éthyl N-hydroxy 7-méthoxy 5-méthylimidazo /1,2-a/ pyrimidine 2-carboxamidine.

On chauffe 2 heures au reflux une suspension comprenant 8 g de 6-éthyl 7-méthoxy 5-méthylimidazo /1,2-a/ pyrimidine 2-carbonitrile préparé comme à l'exemple 1 Stade i, 2,82 g de chlorhydrate d'hydroxylamine et 2,28 g d'hydroxyde de potassium dans 100 cm3 d'éthanol. On refroidit le mélange, filtre le précipité, ajoute de l'eau, agite 15 minutes, filtre, lave à l'eau puis à l'éther, sèche sous pression réduite et obtient 7,96 g de produit attendu. F = 249-250°C.

Stade B : 6-éthyl 7-méthoxy 5-méthyl 2-(5-méthyl 1,2,4-oxadiazol-3-yl) imidazo /1,2-a/ pyrimidine.

On chauffe à 100°C pendant 15 minutes, 1,7 g de produit obtenu au stade A dans 3,55 cm3 de diméthyl acétal de diméthylacétamide. On concentre à sec et purifie par chromatographie sur silice. On recristallise le résidu dans le méthanol et obtient 1, 76 g de produit attendu. F = 176-177°C.

Exemple 3 : 6-éthyl 7-méthoxy 5-méthyl 2-(5-propyl 1,3,4-oxadiazol-2-yl) imidazo /1,2-a/ pyrimidine.

Stade A : 6-éthyl 7-méthoxy 5-méthylimidazo /1,2-a/ pyrimidine 2-carbohydrazide.

On chauffe au reflux pendant 4 heures 10 g de 6-éthyl 7-méthoxy 5-méthylimidazo /1,2-a/ pyrimidine 2-carboxylate d'éthyle (décrit dans la demande anglaise 2 128 989 A) et 20 cm3 d'hydrate d'hydrazine dans 150 cm3 d'éthanol. On évapore en partie l'éthanol, ajoute 100 cm3 d'eau, filtre le précipité obtenu, le lave à l'eau puis à l'éther, le sèche sous pression réduite et obtient 8,15 g de produit attendu.

Stade B : 2-butyryl (6-éthyl 7-méthoxy 5-méthylimidazo /1,2-a/ pyrimidin-2-yl) carbohydrazide.

On chauffe 2 heures au reflux 2 g de produit préparé au stade A, 1,7 cm3 de triéthylamine et 1,96 cm3 d'anhydride butyrique dans 200 cm3 d'éthanol. On refroidit le mélange, filtre le précipité, le lave à l'éthanol puis à l'éther, le sèche et obtient 2,3 g de produit attendu.

Stade C : 6-éthyl 7-méthoxy 5-méthyl 2-(5-propyl 1,3,4-oxadiazol-2-yl) imidazo /1,2-a/ pyrimidine.

On agite pendant 1 heure à 120°C 2,3 g de produit préparé au stade B et 50 g d'acide polyphosphorique. On refroidit le mélange, le verse dans un mélange d'eau et de glace, ajoute du carbonate de sodium jusqu'à neutralité, extrait au chloroforme, sèche et concentre à sec. On purifie le résidu parchromatographie sur silice, recristallise dans l'acétate d'éthyle et obtient 1,62 g de produit attendu. F = 180-181,5°C.

Exemple 4 : 6-éthyl 7-méthoxy 5-méthyl 2-(5-méthyl 1,3,4-thiadiazol-2-yl) imidazo /1,2-a/ pyrimidine.

On opère comme au stade B et C de l'exemple 3 en partant du 6-éthyl 7-méthoxy 5-méthylimidazo /1,2-a/ pyrimidine 2-carbohydrazide et de l'anhydride acétique et obtient le produit attendu.

Exemple 5 : 6-éthyl 7-méthoxy 5-méthyl 2-(5-méthyl 1,3,4-oxadiazol-2-yl) imidazo /1,2-a/ pyrimidine.

On chauffe au reflux pendant 45 minutes 2,67 g de 2-acétyl (6-éthyl 7-méthoxy 5-méthylimidazo /1,2-a/ pyrimidin-2-yl) carbohydrazide préparé comme aux stades A et B de l'exemple 3 mais en utilisant l'anhydride acétique à la place de l'anhydride butyrique et 4,7 g de réactif de Lawesson dans 90 cm3 de toluène. On refroidit le mélange, ajoute 100 cm3 de chloroforme et 100 cm3 d'eau. On lave la phase organique avec une solution aqueuse de bicarbonate de sodium à 5% puis avec une solution aqueuse de soude puis une solution aqueuse saturée de chlorure de sodium, sèche et évapore à sec. On purifie le résidu par chormatographie sur silice, recristallise dans l'acétate d'éthyle et obtient 0,9 g de produit attendu. F = 210,5-212°C.

Exemple 6 : 6-éthyl 7-méthoxy 5-méthyl 2-(5-trifluorométhyl 1,3,4-oxadiazol-2-yl) imidazo /1,2-a/ pyrimidine.

On ajoute sous agitation à température ambiante 1,7 cm3 d'anhydride trifluoroacétique dans une solution comprenant 1,2 g de 6-éthyl 7-méthoxy 5-méthylimidazo /1,2-a/ pyrimidine 2-carbohydrazide préparé comme à l'exemple 3 stade A et 0,8 cm3 de triéthylamine dans 30 cm3 de dichlorométhane. On chauffe le mélange à 45°C pendant 30 minutes, refroidit, lave à l'eau, sèche et concentre à sec. On recristallise le produit dans l'acétate d'éthyle et obtient 1,5 g de produit attendu. F = 210-211°C.

Exemple 7 : 6-éthyl 7-méthoxy 5-méthyl 2-(5 trifluorométhyl 1,2,4-oxadiazol-3-yl) imidazo /1,2-a/ pyrimidine.

On opère comme à l'exemple 6 mais en partant du 6-éthyl N-hydroxy 7-méthoxy 5-méthylimidazo /1,2-a/ pyrimidine 2-carboxamidine préparé comme à l'exemple 2 stade A. On obtient le produit attendu avec un rendement de 95%. F = 197-200°C.

Exemple 8 : 6-éthyl 7-méthoxy 5-méthyl 2-(1,3,4-oxadiazol-2-yl) imidazo /1,2-a/ pyrimidine.

On chauffe 6 heures à 125¤C 2,5 g de 6-éthyl 7-méthoxy 5-méthylimidazo /1,2-a/ pyrimidine 2-carbohydrazide préparé comme à l'exemple 3 stade A en suspension dans 20 cm3 d'orthoformate de triéthyle. On refroidit le mélange, filtre le précipité, le lave à l'éther, le sèche et obtient 2,88 g de N-/(6-éthyl 7-méthoxy 5-méthylimidazo /1,2-a/ pyrimidin-2-yl) carboxamido/ formimidate d'éthyle. On agite 1 heure à 120°C 1,88 g du produit obtenu ci-dessus en suspension dans 50 g d'acide polyphosphorique. On refroidit le mélange, le verse dans de l'eau glacée, neutralise à pH 7 à l'aide de carbonate de sodium, extrait au chloroforme, sépare la phase organique, la sèche et la concentre à sec. On purifie le résidu par chromatographie et obtient après recristallisation dans l'acétate d'éthyle 1,1 g de produit attendu. F = 198,5-199°C.

Exemple 9 : 6-éthyl 2-(5-éthyl 1,3,4-oxadiazol-2-yl) 7-méthoxy 5-méthylimidazo /1,2-a/ pyrimidine.

On opère selon la méthode décrite aux stades B et C de l'exemple 3 en utilisant l'anhydride propionique à la place de l'anhydride butyrique. On obtient le produit attendu avec un rendement de 46%. F = 170-172°C.

En utilisant un mode opératoire identique à celui décrit à l'exemple 3 stades B et C en partant de l'hydrazide de formule XI approprié et de l'anhydride acétique, on a préparé les composés des exemples 10 à 13 :

Exemple 10 : 6-éthyl 5-méthyl 2-(5-méthyl 1,3,4-oxadiazol-2-yl) méthylthioimidazo /1,2-a/ pyrimidine.

Exemple 11 : 6,7,8,9-tétrahydro 5-méthoxy 2-(5-méthyl 1,3,4-oxadiazol-2-yl) imidazo /1,2-a/ quinazoline.

Exemple 12 : 7-méthoxy 5-méthyl 2-(5-méthyl 1,3,4-oxadiazol-2-yl) 6-propylimidazo /1,2-a/ pyrimidine.

Exemple 13 : 7-méthoxy 5-méthyl 2-(5-méthyl 1,3,4-oxadiazol-2-yl) 6-(1-propényl) imidazo /1,2-a/ pyrimidine.

En opérant comme à l'exemple 2 mais en partant du carbonitrile de formule VIII approprié, on a préparé les composés des exemples 14 à 17 :

Exemple 14 : 7-méthoxy 5-méthyl 2-(5-méthyl 1,2,4-oxadiazol-3-yl) 6-propylimidazo /1,2-a/ pyrimidine.

Exemple 15 : 6-éthyl 5-méthyl 2-(5-méthyl 1,2,4-oxadiazol-3-yl) 7-méthylthioimidazo /1,2-a/ pyrimidine.

Exemple 16 : 6-allyl 7-méthoxy 2-(5-méthyl 1,2,4-oxadiazol-3-yl) 5-méthylimidazo /1,2-a/ pyrimidine.

Exemple 17 : 6,7,8,9-tétrahydro 5-méthoxy 2-(5-méthyl 1,2,4-oxadiazol-3-yl) imidazo /1,2-a/ quinazoline.

Le composé de l'exemple 18 a été préparé comme suit :

Exemple 18 : 6-allyl 7-méthoxy 5-méthyl 2-(5-méthyl 1,3,4-oxadiazol-2-yl) imidazo /1,2-a/ pyrimidine.

On chauffe 16 heures à 150-180°C 3,58 g de 6-allyl 7-méthoxy 5-méthylimidazo /1,2-a/ pyrimidine 2-carbohydrazide et 25 cm3 d'orhtoacétate de triéthyle. On refroidit le mélange, concentre à sec, purifie le résidu par chromatographie et obtient 1,47 g de produit attendu F = 186-188¤C.

Les analyses spectométriques, les rendements, les points de fusion et les résultats de la microanalyse sont donnés dans le tableau I ci-après.

TABLEAU I

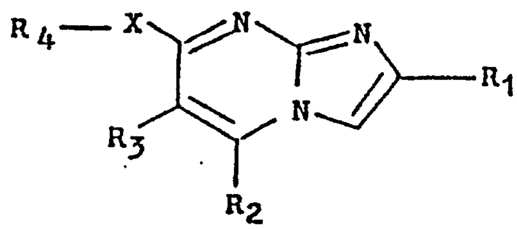

(I)

| Exemple | $R_1$ | $R_2$ | $R_3$ | $R_4$ | X | Rendt (%) | Spectre IR(KBr)$cm^{-1}$ |
|---|---|---|---|---|---|---|---|
| 1(ii) | 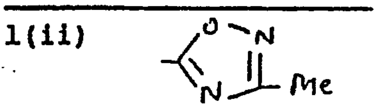 | Me | Et | Me | O | 63 | 3145,1638,1613 |
| 2 | 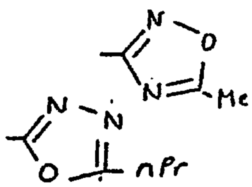 | Me | Et | Me | O | 95 | 3170,1638,1603 |
| 3 | | Me | Et | Me | O | 75 | 3120,2960,2940, 2870,1635,1608 |
| 4 | 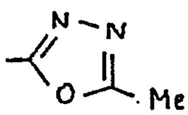 | Me | Et | Me | O | 34 | 3140,2980,2955, 1630 |
| 5 | 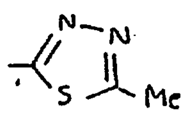 | Me | Et | Me | O | 34 | 3102,2960,2925, 2875,1635 |
| 6 | 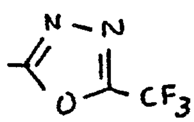 | Me | Et | Me | O | 82 | 3125,2975,2950, 1640,1605 |
| 7 | 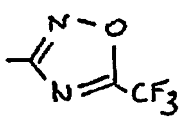 | Me | Et | Me | O | 95 | 3150,2990,2950, 1635,1600 |
| 8 | 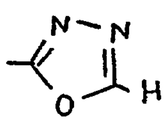 | Me | Et | Me | O | 69 | 3120,2970,2945, 1635,1605 |
| 9 | 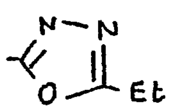 | Me | Et | Me | O | 46 | 3150,2978,2950, 1625 |

TABLEAU I. (Suite)

| Exemple | $R_1$ | $R_2$ | $R_3$ | $R_4$ | X | Rendt. (%) | Spectre IR(KBr)$cm^{-1}$ |
|---|---|---|---|---|---|---|---|
| 10 | 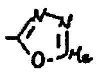 | Me | Et | Me | S | 81 | 3160, 2960, 2930, 1615 |
| 11 | 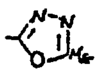 | $-CH_2CH_2CH_2CH_2-$ | | Me | O | 77 | 3175,2990,2940, 2880,1640,1627 |
| 12 | 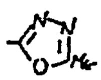 | Me | Pr | Me | O | 93 | 3125,3100,2950, 2930,2870,1635, 1610 |
| 13 | 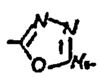 | Me | $CH_3CH=CH-$ | Me | O | 48 | 2955,1630,1615 |
| 14 | 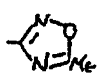 | Me | Pr | Me | O | 87 | 3155,2950,2870, 1636,1600 |
| 15 | 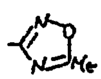 | Me | Et | Me | S | 89 | 2960,2930,1613 |
| 16 | 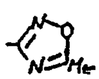 | Me | allyl | Me | O | 80 | 3000,2950, 1635,1600 |
| 17 | 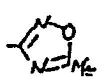 | $-CH_2CH_2CH_2CH_2-$ | | Me | O | 75 | 2945,2880,1643, 1600 |
| 18 | 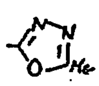 | Me | allyl | Me | O | 34 | 3122,3102,2950, 2925,1635,1610 |

TABLEAU I (Suite)

| Exemple | Fusion | Formule | Poids Moléculaire | C | H | Calculé N | Trouvé S/F |
|---|---|---|---|---|---|---|---|
| 1 (ii) | 204–205 | $C_{13}H_{15}N_5O_2$ | 273.3 | 57.13<br>57.14 | 5.53<br>5.53 | 25.62<br>25.58 | |
| 2 | 176–177 | $C_{13}H_{15}N_5O_2$ | 273.3 | 57.13<br>57.08 | 5.53<br>5.59 | 25.62<br>25.64 | |
| 3 | 180–181.5 | $C_{15}H_{19}N_5O_2$ | 301.3 | 59.79<br>60.07 | 6.35<br>6.33 | 23.24<br>23.26 | |
| 4 | 193.5–195 | $C_{13}H_{15}N_5O_2$ | 273.3 | 57.13<br>57.35 | 5.53<br>5.50 | 25.62<br>25.59 | |
| 5 | 210.5–212 | $C_{13}H_{15}N_5OS$<br>+ $^1/_4$ $H_2O$ | 293.8 | 53.14<br>53.31 | 5.32<br>5.15 | 23.83<br>23.79 | 10.91<br>11.19 |
| 6 | 210–211 | $C_{13}H_{12}N_5O_2F_3$ | 327.3 | 47.71<br>47.56 | 3.70<br>3.73 | 21.40<br>21.44 | 17.42<br>17.44 |
| 7 | 197–200 | $C_{13}H_{12}N_5O_2F_3$ | 327.3 | 47.71<br>47.79 | 3.70<br>3.78 | 21.40<br>21.39 | 17.42<br>17.25 |
| 8 | 198.5–199 | $C_{12}H_{13}N_5O_2$ | 259.3 | 55.59 | 5.05 | 27.01 | |
| 9 | 170–172 | $C_{14}H_{17}N_5O_2$ | 287.3 | 58.53 | 5.97 | 24.37 | |
| 10 | 175–175.5 | $C_{13}H_{15}N_5OS$ | 289.35 | 53.96<br>53.96 | 5.34<br>5.28 | 24.20<br>24.21 | 11.08<br>11.08 |
| 11 | 222–226 | $C_{14}H_{15}N_5O_2$ | 285.31 | 58.94<br>58.92 | 5.30<br>5.34 | 24.55<br>24.60 | |
| 12 | 219–220 | $C_{14}H_{17}N_5O_2$ | 287.33 | 58.53<br>58.56 | 5.97<br>5.99 | 24.37<br>24.43 | |
| 13 | 202–210 | $C_{14}H_{15}N_5O_2$ | 285.31 | 58.94<br>58.64 | 5.30<br>5.36 | 24.55<br>24.15 | |
| 14 | 185–185.5 | $C_{14}H_{17}N_5O_2$ | 287.33 | 58.53<br>58.59 | 5.97<br>5.99 | 24.37<br>24.47 | |
| 15 | 209–210 | $C_{13}H_{15}N_5OS$ | 289.30 | 53.96<br>53.91 | 5.23<br>5.24 | 24.20<br>24.23 | 11.08<br>11.03 |
| 16 | 147–150 | $C_{14}H_{15}N_5O_2$<br>+ $^2/_3$ $H_2O$ | 297.32 | 56.55<br>56.69 | 5.54<br>5.26 | 23.55<br>23.67 | |
| 17 | 218–222 | $C_{14}H_{15}N_5O_2$ | 285.31 | 58.94<br>58.95 | 5.30<br>5.35 | 24.55<br>24.55 | |
| 18 | 186–188 | $C_{14}H_{15}N_5O_2$ | 285.31 | 58.94<br>58.88 | 5.30<br>5.35 | 24.55<br>24.46 | |

Exemple 19 :

On a préparé des comprimés correspondant à la formulation suivante :

- Composé de l'exemple 1 (ii) .......................................... 20 mg
- Excipient q.s. pour un comprimé terminé à .................... 150 mg.

(Détail de l'excipient : lactose, amidon, talc, stéarate de magnésium).

Exemple 20 :

On a préparé des comprimés correspondant à la formulation suivante :

- Composé de l'exemple 4 ............................................ 20 mg
- Excipient q.s. pour un comprimé terminé à .................... 150 mg.

(Détail de l'excipient : lactose, amidon, talc, stéarate de magnésium).

ACTIVITE PHARMACOLOGIQUE

L'étude de l'activité anxiolytique des composés a été effectuée par une méthode selon Geller et Seifter (Psychopharmacologia, 1960, I, 482) modifiée.

Les valeurs données dans le tableau II sont les doses efficaces minimum auxquelles il est observé un accroissement des chocs par rapport aux témoins (DEM mg/kg po).

TABLEAU II

| Exemple | TEST DE GELLER (DEM mg/kg p.o.) |
|---|---|
| 1 | 5 |
| 2 | 5 |
| 3 | 10–50 |
| 4 | 2 |
| 5 | 10 |
| 6 | 10–50 |
| 7 | 2 |
| 8 | >50 |
| 9 | 50 |
| 10 | 5 |
| 11 | 50 |
| 12 | 10 |
| 13 | 50 |
| 14 | 5 |
| 15 | 2 |
| 16 | 10 |
| 17 | 5 |
| 18 | 10 |

**Revendications**

1. Imidazo[1,2-a]pyrimidines, ainsi que leurs sels d'addition avec les acides minéraux ou organiques, caractérisées en ce qu'elles répondent à la formule générale (I):

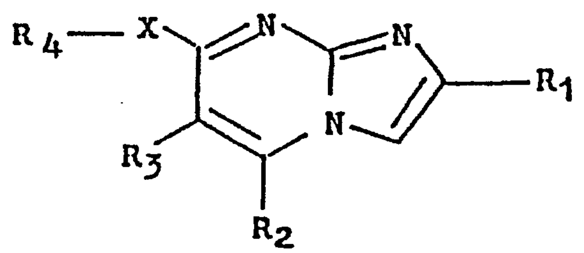

(I)

dans laquelle:

– $R_1$ représente un groupement 1,2,4-oxadiazol-5-yl ou 1,3,4-thiadiazol-2-yl éventuellement substitué par un radical alkyl renfermant de 1 à 3 atomes de carbone ou un radical alkényl renfermant de 2 à 5 atomes de carbone ou un groupement 1,2,4-oxadiazol-3-yl ou 1,3,4-oxadiazol-2-yl éventuellement substitué par un radical alkyl renfermant de 1 à 3 atomes de carbone lui-même éventuellement substitué par un ou plusieurs atomes de fluor, ou par un radical alkényle renfermant de 2 à 5 atomes de carbone,

– $R_2$, $R_3$, identiques ou différents, représentent un atome d'hydrogène, un radical alkyl renfermant de 1 à 3 atomes de carbone, un radical alkényl renfermant de 2 à 5 atomes de carbone, ou $R_2$ et $R_3$ forment ensemble un radical alkylène renfermant de 3 à 5 atomes de carbone,

– X représente un atome d'oxygène ou de soufre,

– $R_4$ représente un radical alkyl renfermant de 1 à 3 atomes de carbone.

2. Imidazo[1,2-a]pyrimidines telles que définies par la formule (I) de la revendication 1, ainsi que leurs sels, caractérisées en ce que dans ladite formule (I), $R_1$ représente un radical 5-méthyl 1,3,4-oxadiazol-5-yl, 5-propyl 1,3,4-oxadiazol-2-yl, 5-trifluorométhyl 1,2,4-oxadiazol-3-yl, 5-trifluorométhyl 1,3,4-oxadiazol-2-yl ou 5-méthyl 1,3,4-thiadiazol-2-yl.

3. Imidazo[1,2-a]pyrimidines telles que définies par la formule (I) de la revendication 1, ainsi que leurs sels, caractérisées en ce que dans ladite formule (I), $R_1$ représente un radical 3-méthyl 1,2,4-oxadiazol-5-yl, un radical 5-méthyl 1,2,4-oxadiazol-3-yl, un radical 5-propyl 1,3,4-oxadiazol-2-yl, un radical 5-trifluorométhyl 1,2,4-oxadiazol-3-yl, un radical 5-méthyl 1,3,4-oxadiazol-2-yl ou un radical 5-méthyl 1,3,4-thiadiazol-2-yl.

4. Imidazo[1,2-a]pyrimidines telles que défninies à l'une quelconque des revendications 1, 2 ou 3, ainsi que leurs sels, caractérisées en ce que dans la formule (I), $R_2$ et $R_3$ représentent un radical alkyl renfermant de 1 à 3 atomes de carbone et X représente un atome d'oxygène.

5. L'un quelconque des dérivés répondant à la formule (I) de la revendication 1, dont les noms suivent:
- la 6-éthyl 7-méthoxy 5-méthyl 2-(3-méthyl 1,2,4-oxadiazol-5-yl) imidazo[1,2-a]pyrimidine et ses sels;
- la 6-éthyl 7-méthoxy 5-méthyl 2-(5-méthyl 1,2,4-oxadiazol-3-yl) imidazo[1,2-a]pyrimidine et ses sels;
- la 6-éthyl 7-méthoxy 5-méthyl 2-(5-trifluorométhyl 1,2,4-oxadiazol-3-yl) imidazo[1,2-a]pyrimidine et ses sels;
- la 6-éthyl 7-méthoxy 5-méthyl 2-(5-méthyl 1,3,4-oxadiazol-2-yl) imidazo[1,2-a]pyrimidine et ses sels;
- la 6-éthyl 5-méthyl 2-(5-méthyl 1,3,4-oxadiazol-2-yl) 7-méthylthioimidazo[1,2-a]pyrimidine et ses sels;
- la 7-méthoxy 5-méthyl 2-(5-méthyl 1,2,4-oxadiazol-3-yl) 6-propylimidazo[1,2-a]pyrimidine et ses sels;
- la 6-éthyl 5-méthyl 2-(5-méthyl 1,2,4-oxadiazol-3-yl) 7-méthylthioimidazo[1,2-a]pyrimidine et ses sels;
- la 6,7,8,9-tétrahydro 5-méthoxy 2-(5-méthyl 1,2,4-oxadiazol-3-yl) imidazo[1,2-a]quinazoline et ses sels.

6. Procédé de préparation des imidazo[1,2-a]pyrimidines telles que définies par la formule (I) de la revendication 1, dans laquelle $R_1$ représente un radical 1,2,4-oxadiazol-5-yl ainsi que de leurs sels, caractérisé en ce que l'on fait réagir un produit de formule (II):

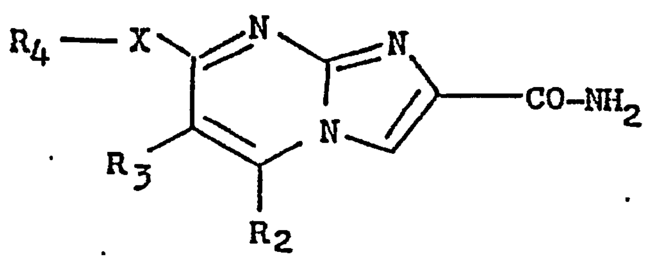

(II)

dans laquelle $R_2$, $R_3$, $R_4$ et X ont la signification déjà indiquée, avec un produit de formule (III):

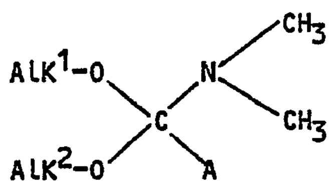

(III)

dans laquelle $ALK^1$ et $ALK^2$ représentent chacun un radical alkyl renfermant de 1 à 3 atomes de carbone et A représente un atome d'hydrogène, un radical alkyl renfermant de 1 à 3 atomes de carbone ou un radical alkényl renfermant de 2 à 5 atomes de carbone, pour obtenir un produit de formule (IV):

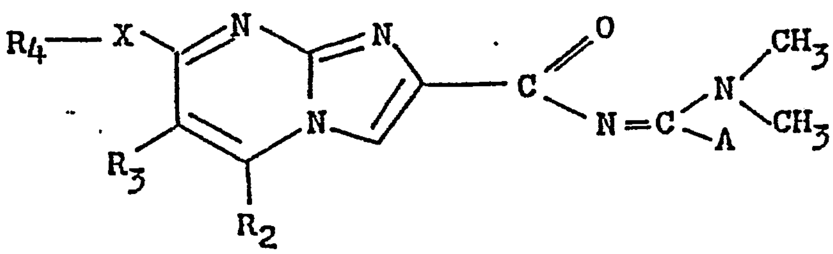

(IV)

dans laquelle $R_2$, $R_3$, $R_4$, X et A ont la signification déjà indiquée, puis fait réagir ce dernier avec le chlorhydrate d'hydroxylamine, pour obtenir le produit de formule ($I_A$):

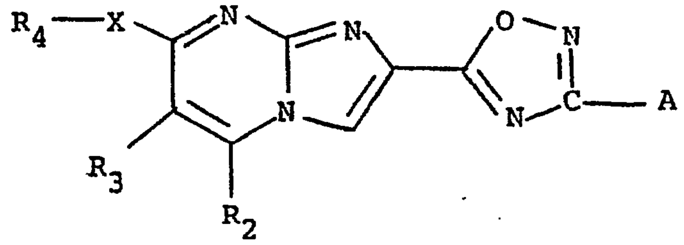

($I_A$)

recherché, dans laquelle $R_2$, $R_3$, $R_4$, X et A ont la signification déjà indiquée, puis le cas échéant, salifie le produit de formule (I) ainsi obtenu.

7. Procédé de préparation selon la revendication 6, caractérisé en ce que:

a) la réaction du produit de formule (II) avec le produit de formule (III) est effectuée par chauffage du milieu réactionnel;

b) la réaction du produit de formule (IV) avec le chlorhydrate d'hydroxylamine est effectuée en présence d'une base au sein d'un solvant organique en présence d'un cosolvant.

8. Procédé de préparation des imidazo[1,2-a]pyrimidines telles que définies par la formule (I) de la revendication 1, dans laquelle $R_1$ représente un radical 1,2,4-oxadiazol-3-yl, ainsi que de leurs sels, caractérisé en ce que l'on fait réagir un produit de formule (V) :

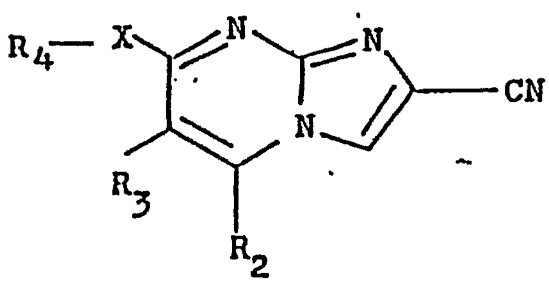

(V)

dans laquelle $R_2$, $R_3$, $R_4$ et X ont la signification déjà indiquée, avec le chlorhydrate d'hydroxylamine, pour obtenir un produit de formule (VI) :

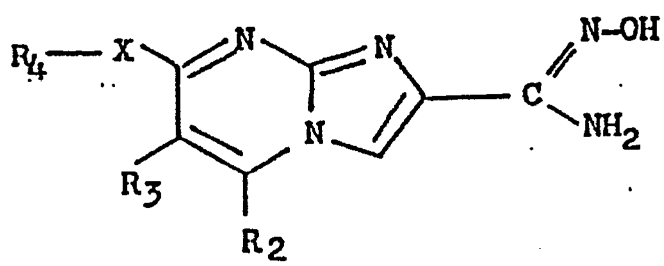

(VI)

dans laquelle $R_2$, $R_3$, $R_4$ et X ont la signification déjà indiquée, puis fait réagir ce dernier avec un dérivé fonctionnel d'un acide de formule (IX) :

D-COOH (IX)

dans laquelle D représente un atome d'hydrogène, un radical alkyl renfermant de 1 à 3 atomes de carbone éventuellement substitué par un ou plusieurs atomes de fluor ou un radical alkényl renfermant de 2 à 5 atomes de carbone pour obtenir un produit de formule ($I_B$) :

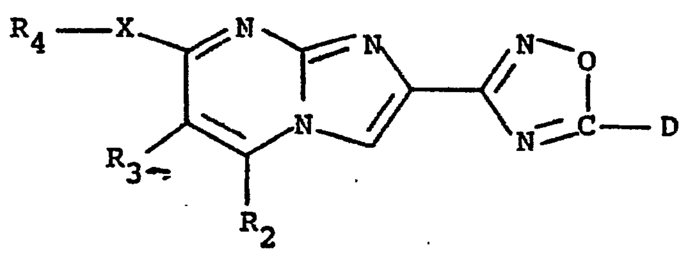

($I_B$)

dans laquelle $R_2$, $R_3$, $R_4$, X et D ont la signification déjà indiquée, puis le cas échéant, salifie le produit de formule (I) ainsi obtenu.

9. Procédé de préparation selon la revendication 8, caractérisé en ce que:
a) la réaction du produit de formule (V) avec le chlorhydrate d'hydroxylamine est effectuée par chauffage au reflux du mélange réactionnel au sein d'un solvant organique, en présence d'une base;
b) le dérivé fonctionnel de l'acide de formule (IX)

D–COOH (IX)

est un anhydride d'acide ou un amide acétal de formule (III'):

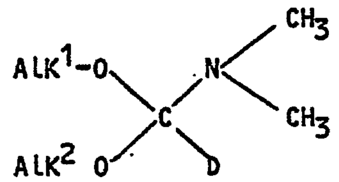

(III')

dans laquelle ALK[1] et ALK[2] ont les significations précédentes;
c) la réaction du produit de formule (VI) avec le dérivé fonctionnel de l'acide D–COOH est effectuée par chauffage du milieu réactionnel;
d) la réaction du produit de formule (VI) avec le dérivé fonctionnel de l'acide D–COOH est effectuée en présence d'une base au sein d'un solvant organique au reflux si le dérivé réactif est un agent déshydratant fort.

10. Procédé de préparation des imidazo[1,2-a]pyrimidines telles que définies par la formule (I) de la revendication 1, dans laquelle $R_1$ représente un radical 1,3,4-oxadiazol-2-yl, ainsi que de leurs sels, caractérisé en ce que l'on fait réagir un produit de formule (VII):

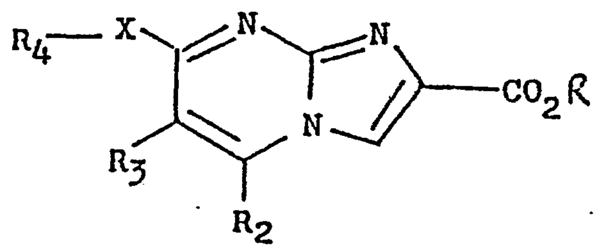

(VII)

dans laquelle $R_2$, $R_3$, $R_4$ et X ont la signification déjà indiquée et R représente le reste d'un groupement ester avec de l'hydrate d'hydrazine pour obtenir un produit de formule (VIII) :

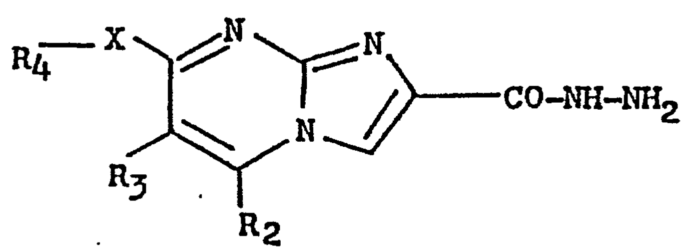

(VIII)

dans laquelle $R_2$, $R_3$, $R_4$ et X ont la signification déjà indiquée, puis fait réagir ce dernier avec soit un anhydride ou un chlorure d'acide de formule (IX) :

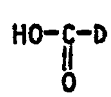

(IX)

dans laquelle D a la signification déjà indiquée, pour obtenir un produit de formule (X) :

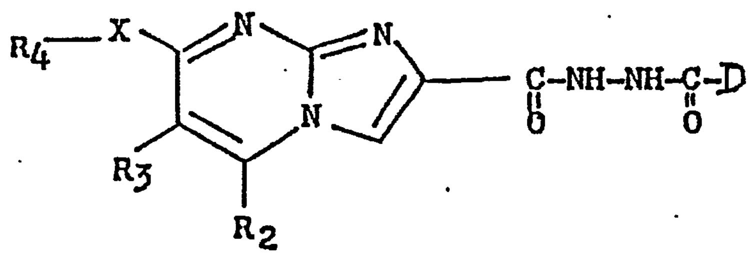

(X)

soit avec un orthoester de formule

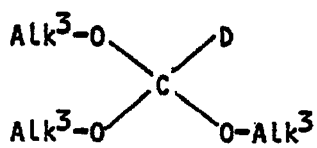

dans lequel $Alk^3$ représente un radical alkyle renfermant de 1 à 3 atomes de carbone pour obtenir un produit de formule (XI) :

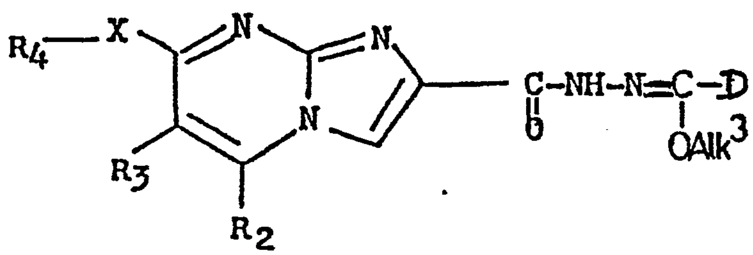

(XI)

dans laquelle $R_2$, $R_3$, $R_4$, X et D ont la signification déjà indiquée et $Alk^3$ a la signification précédente puis cyclise par chauffage le produit de formule (X) ou (XI), pour obtenir un produit de formule (Ic):

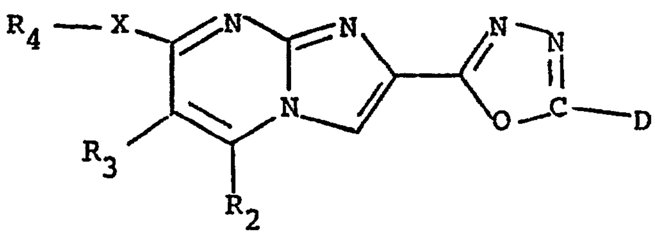

($I_C$)

dans laquelle $R_2$, $R_3$, $R_4$, X et D ont la signification déjà indiquée, puis le cas échéant, salifie le produit de formule (I) ainsi obtenu.

11. Procédé de préparation selon la revendication 10, caractérisé en ce que:

a) le reste du groupement ester que représente R est un radical alkyle renfermant de 1 à 3 atomes de carbone;

b) la réaction du produit de formule (VII) avec l'hydrate d'hydrazine est effectuée par chauffage au reflux du mélange réactionnel au sein d'un solvant organique;

c) quand le dérivé réactif que l'on fait agir sur le produit de formule (VIII) est agent déshydratant fort, le produit de formule (X) ou (XI) est préparé in situ dans le milieu réactionnel et la cyclisation est effectuée en présence d'une base au sein d'un solvant organique à une température comprise entre 25° et 45°C;

d) quand le dérivé réactif que l'on fait réagir sur le produit de formule (VIII) n'est pas un agent déshydratant fort, le produit de formule (X) ou (XI) est isolé et la réaction de cyclisation est effectuée par chauffage du milieu réactionnel en présence de chlorure de phosphonyl ou de préférence en présence d'un acide fort.

12. Procédé de préparation des imidazo[1,2-a]pyrimidines telles que définies par la formule (I) de la revendication 1, dans laquelle $R_1$ représente un radical 1,3,4-thiadiazol-2-yl, ainsi que de leurs sels, caractérisé en ce que l'on fait réagir un produit de formule (VIII):

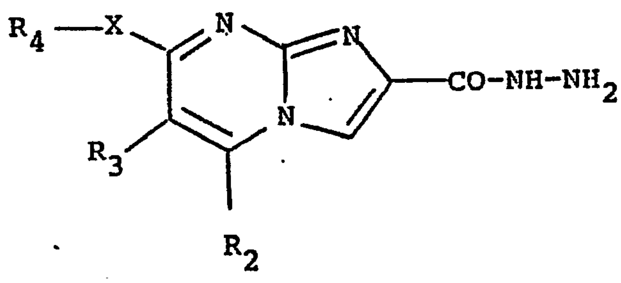

(VIII)

dans laquelle $R_2$, $R_3$, $R_4$ et X ont la signification déjà indiquée avec le dérivé fonctionnel d'un acide de formule (IX') :

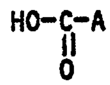

(IX')

dans laquelle A a la signification déjà indiquée, pour obtenir un produit de formule (XII) :

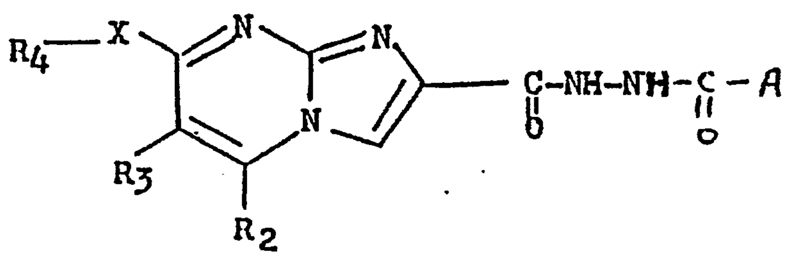

(XII)

dans laquelle $R_2$, $R_3$ $R_4$ X et A ont la signification déjà indiquée, puis fait réagir ce dernier avec le réactif de Lawesson au sein d'un solvant organique pour obtenir un produit de formule ($I_D$) :

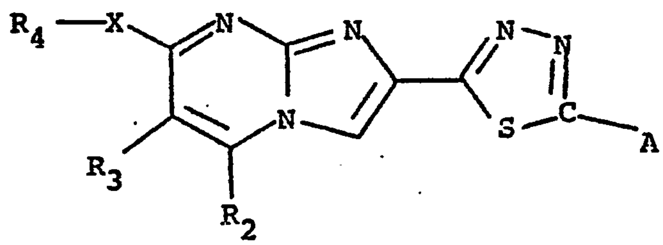

($I_D$)

dans laquelle $R_2$, $R_3$, $R_4$, X et A ont la signification déjà indiquée, puis le cas échéant, salifie le produit de formule (I) ainsi obtenu.

13.- Procédé de préparation selon la revendication 12, caractérisé en ce que :

- la réaction avec le réactif de Lawesson ou (2,4-bis 4-méthoxyphényl) 1,3-dithia 2,4-diphosphétane 2,4-disulphide) de formule :

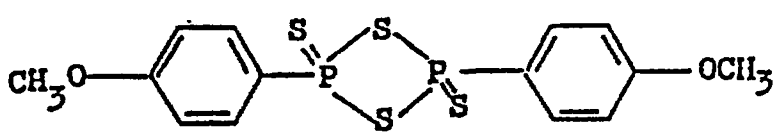

est effectuée au sein d'un solvant organique au reflux du mélange réactionnel.

14.- Médicaments, caractérisés en ce qu'ils sont constitués par les imidazo /1,2-a/ pyrimidines telles que définies par la formule (I) de la revendication 1, ainsi que par leurs sels pharmaceutiquement acceptables.

15.- Médicaments, caractérisés en ce qu'ils sont constitués par les imidazo /1,2-a/ pyrimidines, telles que définies à l'une quelconque des revendications 2, 3 ou 4, ainsi que par leurs sels pharmaceutiquement acceptables.

16.- Médicaments, caractérisés en ce qu'ils sont constitués par les imidazo /1,2-a/ pyrimidines, telles que définies à la revendication 5.

17.- Compositions pharmaceutiques, caractérisées en ce qu'elles renferment à titre de principe actif, l'un au moins des médicaments tels que définis à l'une quelconque des revendications 14 à 16.

**Claims**

1. Imidazo-[1,2-a]-pyrimidines, as well as their addition salts with mineral or organic acids, characterized in that they correspond to the general formula (I):

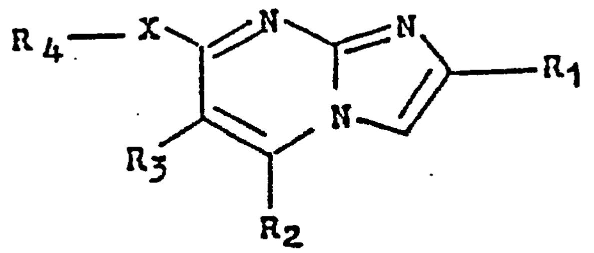

(I)

in which:

– $R_1$ represents a 1,2,4-oxadiazol-5-yl or 1,3,4-thiadiazol-2-yl group optionally substituted by an alkyl radical containing 1 to 3 carbon atoms or an alkenyl radical containing 2 to 5 carbon atoms, or a 1,2,4-oxadiazol-3-yl or 1,3,4-oxadiazol-2-yl group optionally substituted by an alkyl radical containing 1 to 3 carbon atoms, itself optionally substituted by one or more fluorine atoms, or by an alkenyl radical containing 2 to 5 carbon atoms,

– $R_2$, $R_3$, identical or different, represent a hydrogen atom, an alkyl radical containing 1 to 3 carbon atoms, an alkenyl radical containing 2 to 5 carbon atoms, or $R_2$ and $R_3$ together form an alkylene radical containing 3 to 5 carbon atoms,

– X represents an oxygen or sulphur atom,

– $R_4$ represents an alkyl radical containing 1 to 3 carbon atoms.

2. Imidazo-[1,2-a]-pyrimidines as defined by formula (I) of claim 1, as well as their salts, characterized in that in the said formula (I), $R_1$ represents one of the following radicals: 5-methyl-1,3,4-oxadiazol-5-yl, 5-propyl-1,3,4-oxadiazol-2-yl, 5-trifluoromethyl-1,2,4-oxadiazol-3-yl, 5-trifluoromethyl-1,3,4-oxadiazol-2-yl or 5-methyl-1,3,4-thiadiazol-2-yl.

3. Imidazo-[1,2-a]-pyrimidines as defined by formula (I) of claim 1, as well as their salts, characterized in that in the said formula (I) $R_1$ represents a 3-methyl-1,2,4-oxadiazol-5-yl radical, a 5-methyl-1,2,4-oxadiazol-3-yl radical, a 5-propyl-1,3,4-oxadiazol-2-yl radical, a 5-trifluoromethyl-1,2,4-oxadiazol-3-yl radical, a 5-methyl-1,3,4-oxadiazol-2-yl radical or a 5-methyl-1,3,4-thiadiazol-2-yl radical.

4. Imidazo-[1,2-a]-pyrimidines as defined in any one of claims 1, 2 or 3, as well as their salts, characterized in that in the formula (I), $R_2$ and $R_3$ represent an alkyl radical containing 1 to 3 carbon atoms and X represents an oxygen atom.

5. Any one of the derivatives corresponding to the formula (I) of claim 1, of which the names follow:

– 6-ethyl-7-methoxy-5-methyl-2-(3-methyl-1,2-oxadiazol-5-yl)-imidazo-[1,2-a]-pyrimidine and its salts;

– 6-ethyl-7-methoxy-5-methyl-2-(5-methyl-1,2,4-oxadiazol-3-yl)-imidazo-[1,2-a]-pyrimidine and its salts;

– 6-ethyl-7-methoxy-5-methyl-2-(5-trifluoromethyl-1,2,4-oxadiazol-3-yl)-imidazo-[1,2-a]-pyrimidine and its salts;

– 6-ethyl-7-methoxy-5-methyl-2-(5-methyl-1,3,4-oxadiazol-2-yl)-imidazo-[1,2-a]-pyrimidine and its salts;

– 6-ethyl-5-methyl-2-(5-methyl-1,3,4-oxadiazol-2-yl)-7-methylthio-imidazo-[1,2-a]-pyrimidine and its salts;

– 7-methoxy-5-methyl-2-(5-methyl-1,2,4-oxadiazol-3-yl)-propyl-imidazo-[1,2-a]-pyrimidine and its salts;

– 6-ethyl-5-methyl-2-(5-methyl-1,2,4-oxadiazol-3-yl)-7-methylthio-imidazo-[1,2-a]-pyrimidine and its salts;

– 6,7,8,9-tetrahydro-5-methoxy-2-(5-methyl-1,2,4-oxadiazol-3-yl)-imidazo-[1,2-a]-quinazoline and its salts.

6. Process for the preparation of imidazo-[1,2-a]-pyrimidines as defined by formula (I) of claim 1, in which $R_1$ represents a 1,2,4-oxadiazol-5-yl radical, as well as of their salts, characterized in that a product of formula (II):

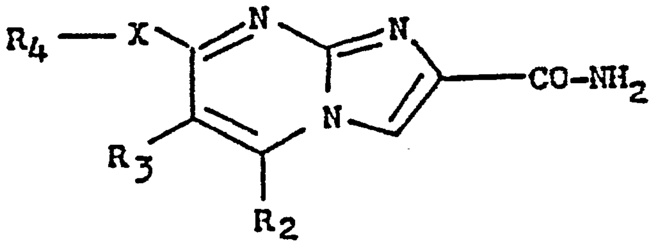

(II)

in which $R_2$, $R_3$, $R_4$ and X have the meaning already indicated, is reacted with a product of formula (III):

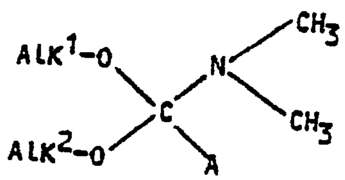

(III)

in which $Alk^1$ and $Alk^2$ each represents an alkyl radical containing 1 to 3 carbon atoms and A represents a hydrogen atom, an alkyl radical containing 1 to 3 carbon atoms or an alkenyl radical containing 2 to 5 carbon atoms, so as to obtain a product of formula (IV):

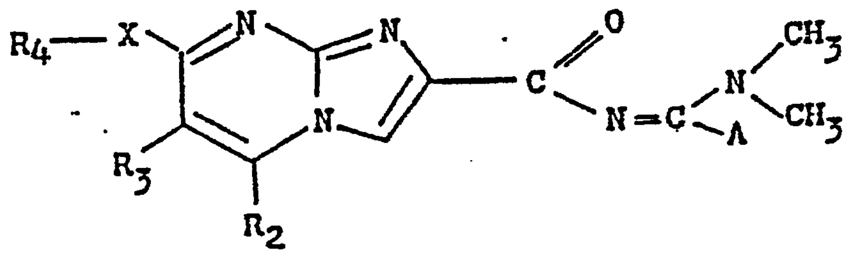

(IV)

in which $R_2$, $R_3$, $R_4$, X and A have the meaning already indicated, then this latter is reacted with hydroxylamine hydrochloride, so as to obtain the sought product of formula ($I_A$):

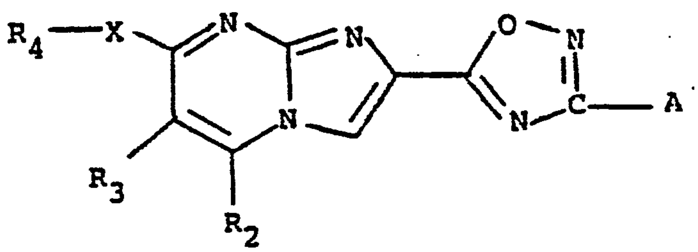

($I_A$)

in which $R_2$, $R_3$, $R_4$, X and A have the meaning already indicated, then if appropriate, the product of formula (I) thus obtained is salified.

7. Preparation process according to claim 6, characterized in that:

a) the reaction of the product of formula (II) with the product of formula (III) is carried out by heating of the reaction medium;

b) the reaction of the product of formula (IV) with the hydroxylamine hydrochloride is carried out in the presence of a base in an organic solvent in the presence of a co-solvent.

8. Process for the preparation of imidazo-[1,2-a]-pyrimidines as defined by formula (I) of claim 1, in which $R_1$ represents a 1,2,4-oxadiazol-3-yl radical, as well as their salts, characterized in that a product of formula (V):

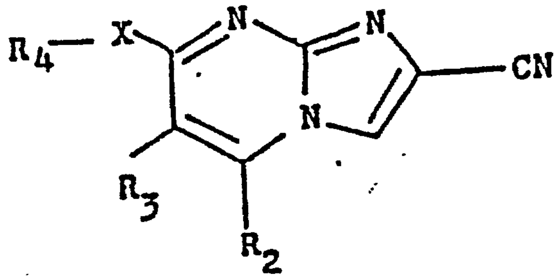

(V)

in which $R_2$, $R_3$, $R_4$ and X have the meaning already indicated, is reacted with hydroxylamine hydrochloride, so as to obtain a product of formula (VI):

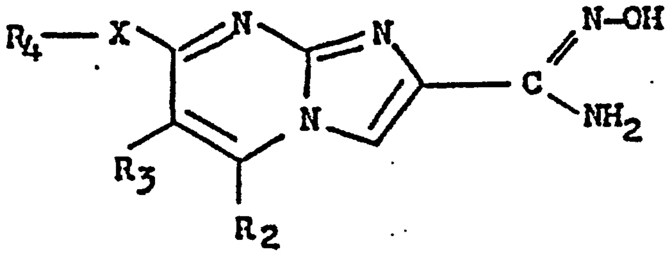

(VI)

in which $R_2$, $R_3$, $R_4$ and X have the meaning already indicated, then this latter is made to react with a functional derivative of an acid of formula (IX):

D–COOH (IX)

in which D represents a hydrogen atom, an alkyl radical containing 1 to 3 carbon atoms optionally substituted by one or more fluorine atoms or an alkenyl radical containing 2 to 5 carbon atoms, so as to obtain a product of formula ($I_B$):

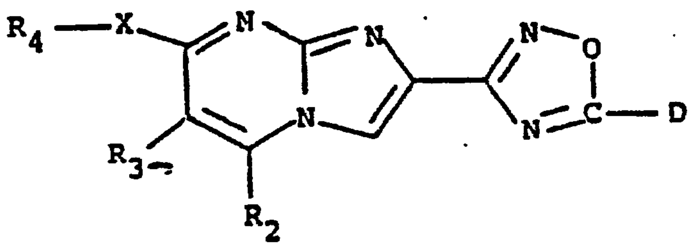

($I_B$)

in which $R_2$, $R_3$, $R_4$, X and D have the meaning already indicated, then if appropriate, the product of formula (I) thus obtained is salified.

9. Preparation process according to claim 8, characterized in that:

a) the reaction of the product of formula (V) with the hydroxylamine hydrochloride is carried out by heating the reaction mixture at reflux in an organic solvent, in the presence of a base;

b) the functional derivative of the acid of formula (IX):

D–COOH (IX)

is an acid anhydride or an acetal amide of formula (III'):

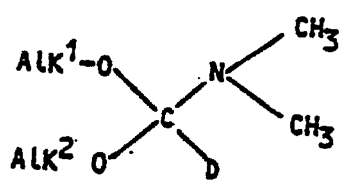

(III')

in which $Alk^1$ and $Alk^2$ have the previous meanings;

c) the reaction of the product of formula (VI) with the functional derivative of the D–COOH acid is carried out by heating the reaction medium;

d) the reaction of the product of formula (VI) with the functional derivative of the D–COOH acid is carried out in the presence of a base in an organic solvent at reflux if the reactive derivative is a strong dehydrating agent.

10. Process for the preparation of imidazo-[1,2-a]-pyrimidines as defined by formula (I) of claim 1, in which $R_1$ represents a 1,3,4-oxadiazol-2-yl radical, as well as of their salts, characterized in that a product of formula (VII):

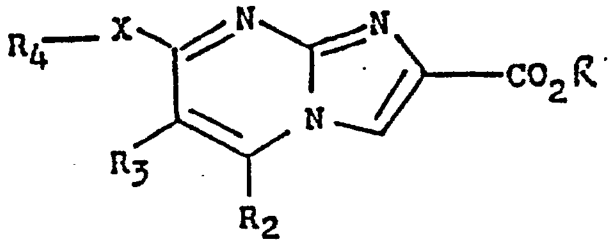

(VII)

in which $R_2$, $R_3$, $R_4$ and X have the meaning already indicated and R represents the residue of an ester group, is reacted with hydrazine hydrate, so as to obtain a product of formula (VIII):

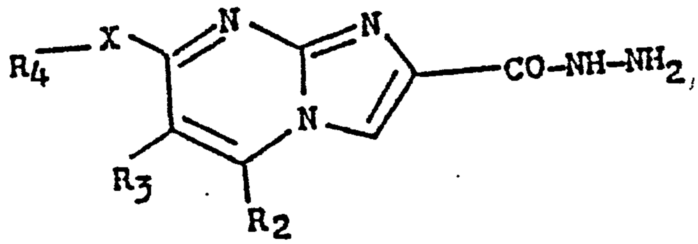

(VIII)

in which $R_2$, $R_3$, $R_4$ and X have the meaning already indicated, then this latter is reacted either with an anhydride or an acid chloride of formula (IX):

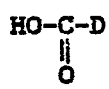

(IX)

in which D has the meaning already indicated, so as to obtain a product of formula (X):

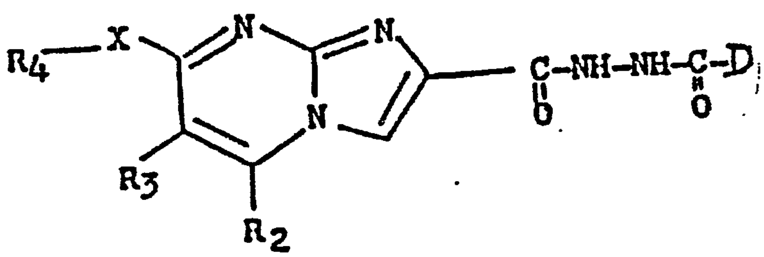

(X)

or with an orthoester of formula:

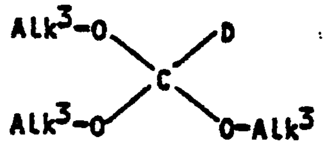

in which $Alk^3$ represents an alkyl radical containing 1 to 3 carbon atoms, so as to obtain a product of formula (XI):

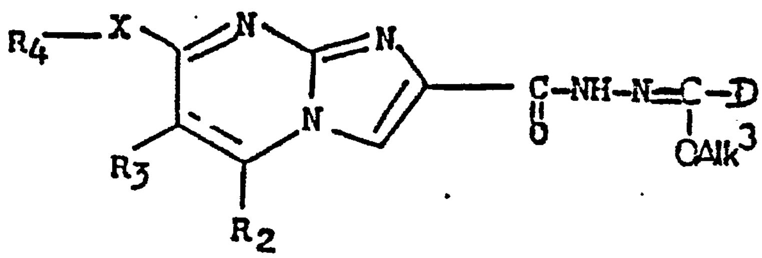

(XI)

in which $R_2$, $R_3$, $R_4$, X and D have the meaning already indicated and $Alk^3$ has the previous meaning, then the product of formula (X) or (XI) is cyclized by heating, so as to obtain a product of formula ($I_C$):

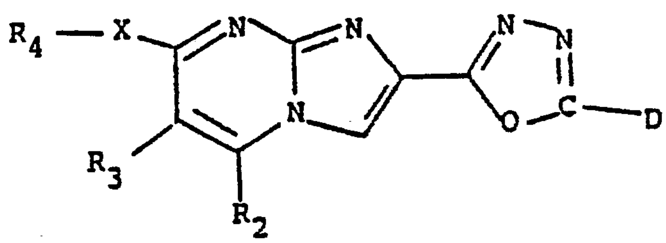

($I_C$)

in which $R_2$, $R_3$, $R_4$, X and D have the meaning already indicated, then if appropriate, the product of formula (I) thus obtained is salified.

11. Preparation process according to claim 10, characterized in that:

a) the residue of the ester group which R represents is an alkyl radical containing 1 to 3 carbon atoms;

b) the reaction of the product of formula (VII) with the hydrazine hydrate is carried out by heating the reaction mixture at reflux in an organic solvent;

c) when the reactive derivative which is reacted on the product of formula (VIII) is a strong dehydrating agent, the product of formula (X) or (XI) is prepared in situ in the reaction medium and the cyclization is carried out in the presence of a base in an organic solvent at a temperature between 25° and 45°C;

d) when the reactive derivative which is reacted with the product of formula (VIII) is not a strong dehydrating agent, the product of formula (X) or (XI) is isolated and the cyclization reaction is carried out by heating the reaction medium in the presence of phosphonyl chloride or preferably in the presence of a strong acid.

12. Process for the preparation of imidazo-[1,2-a]-pyrimidines as defined by formula (I) of claim 1, in which $R_1$ represents a 1,3,4-thiadiazol-2-yl radical, as well as of their salts, characterized in that a product of formula (VIII):

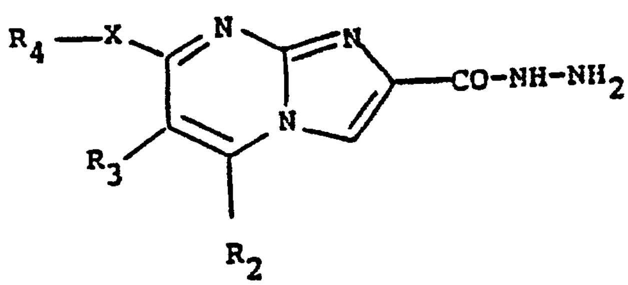

(VIII)

in which $R_2$, $R_3$, $R_4$ and X have the meaning already indicated, is reacted with the functional derivative of an acid of formula (IX'):

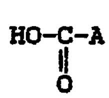

(IX')

in which A has the meaning already indicated, so as to obtain a product of formula (XII):

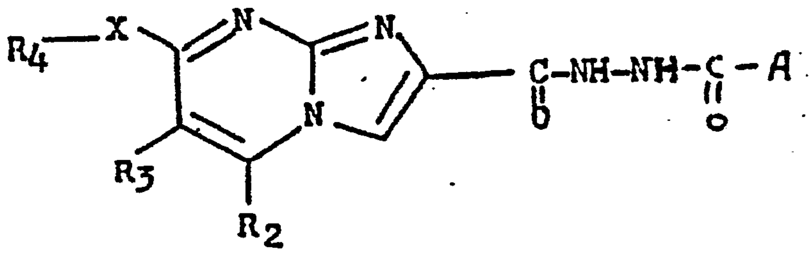

(XII)

in which $R_2$, $R_3$, $R_4$, X and A have the meaning already indicated, then this latter is reacted with Lawesson's reagent in an organic solvent, so as to obtain a product of formula ($I_D$):

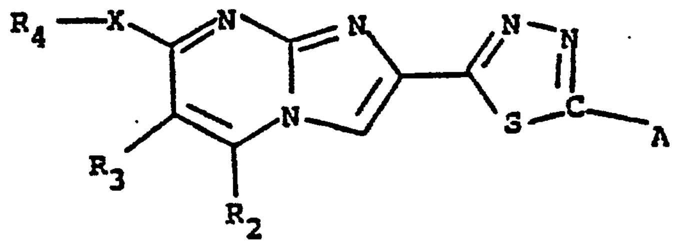

($I_D$)

in which $R_2$, $R_3$, $R_4$, X and A have the meaning already indicated, then if appropriate, the product of formula (I) thus obtained is salified.

13. Preparation process according to claim 12, characterized in that:

– the reaction with Lawesson's reagent or (2,4-bis-4-methoxyphenyl)-1,3-dithia-2,4-diphosphetane-2,4-disulphide) of formula:

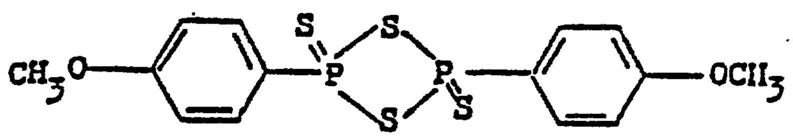

is carried out in an organic solvent at reflux of the reaction mixture.

14. Medicaments, characterized in that they are constituted by imidazo-[1,2-a]-pyrimidines as defined by formula (I) of claim 1 as well as their pharmaceutically acceptable salts.

15. Medicaments, characterized in that they are constituted by imidazo-[1,2-a]-pyrimidines, as defined in any one of claims 2, 3, or 4, as well as by their pharmaceutically acceptable salts.

16. Medicaments, characterized in that they are constituted by imidazo-[1,2-a]-pyrimidines, as defined in claim 5.

17. Pharmaceutical compositions, characterized in that they contain as active principle, at least one of the medicaments as defined in any one of claims 14 to 16.

**Patentansprüche**

1. Imidazo-[1,2-a]-pyrimidine sowie deren Additionssalze mit Mineral- oder organischen Säuren, dadurch gekennzeichnet, daß sie der allgemeinen Formel (I):

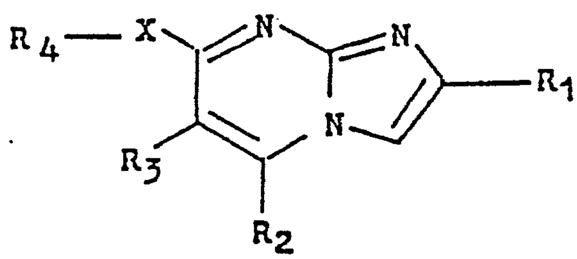

(I)

entsprechen, worin:

– $R_1$ eine 1,2,4-Oxadiazol-5-yl- oder 1,3,4-Thiadiazol-2-yl-Gruppe, die gegebenenfalls durch einen Alkylrest mit 1 bis 3 Kohlenstoffatomen oder einen Alkenylrest mit 2 bis 5 Kohlenstoffatomen substituiert ist, oder eine 1,2,4-Oxadiazol-3-yl- oder 1,3,4-Oxadiazol-2-yl-Gruppe, die gegebenenfalls durch einen Alkylrest mit 1 bis 3 Kohlenstoffatomen, der seinerseits durch ein oder mehrere Fluoratome substituiert sein kann, oder durch einen Alkenylrest mit 2 bis 5 Kohlenstoffatomen substituiert ist, bedeutet,

– $R_2$ und $R_3$, die gleich oder voneinander verschieden sind, für ein Wasserstoffatom, einen Alkylrest mit 1 bis 3 Kohlenstoffatomen oder einen Alkenylrest mit 2 bis 5 Kohlenstoffatomen stehen, oder $R_2$ und $R_3$ gemeinsam einen Alkylenrest mit 3 bis 5 Kohlenstoffatomen bilden,

– X ein Sauerstoff- oder Schwefelatom bedeutet, und

– $R_4$ eine Alkylgruppe mit 1 bis 3 Kohlenstoffatomen wiedergibt.

2. Imidazo-[1,2-a]-pyrimidine der Formel (I) gemäß Anspruch 1 sowie deren Salze, dadurch gekennzeichnet, daß in der Formel (I) $R_1$ für einen 5-Methyl-1,3,4-oxadiazol-5-yl-, 5-Propyl-1,3,4-oxadiazol-2-yl-, 5-Trifluormethyl-1,2,4-oxadiazol-3-yl-, 5-Trifluormethyl-1,3,4-oxadiazol-2-yl- oder 5-Methyl-1,3,4-Thiadiazol-2-yl-Rest steht.

3. Imidazo-[1,2-a]-pyrimidine der Formel (I) gemäß Anspruch 1 sowie deren Salze, dadurch gekennzeichnet, daß in der Formel (I) $R_1$ für einen 3-Methyl-1,2,4-oxadiazol-5-yl-Rest, einen 5-Methyl-1,2,4-oxadiazol-3-yl-Rest, einen 5-Propyl-1,3,4-oxadiazol-2-yl-Rest, einen 5-Trifluormethyl-1,2,4-oxadiazol-3-yl-Rest, einen 5-Methyl-1,3,4-oxadiazol-2-yl-Rest oder einen 5-Methyl-1,3,4-thiadiazol-2-yl-Rest steht.

4. Imidazo-[1,2-a]-pyrimidine gemäß einem der Ansprüche 1, 2 oder 3 sowie deren Salze, dadurch gekennzeichnet, daß in der Formel (I) $R_2$ und $R_3$ für einen Alkylrest mit 1 bis 3 Kohlenstoffatomen stehen und X ein Sauerstoffatom bedeutet.

5. Derivate der Formel (I) gemäß Anspruch 1 mit den folgenden Bezeichnungen:

– 6-Ethyl-7-methoxy-5-methyl-2-(3-methyl-1,2,4-oxadiazol-5-yl)-imidazo-[1,2-a]-pyrimidin und dessen Salze;

– 6-Ethyl-7-methoxy-5-methyl-2-(5-methyl-1,2,4-oxadiazol-3-yl)-imidazo-[1,2-a]-pyrimidin und dessen Salze;

– 6-Ethyl-7-methoxy-5-methyl-2-(5-trifluormethyl-1,2,4-oxadiazol-3-yl)-imidazo-[1,2-a]-pyrimidin und dessen Salze;

– 6-Ethyl-7-methoxy-5-methyl-2-(5-methyl-1,3,4-oxadiazol-2-yl)-imidazo-[1,2-a]-pyrimidin und dessen Salze;

– 6-Ethyl-5-methyl-2-(5-methyl-1,3,4-oxadiazol-2-yl)-7-methylthioimidazo-[1,2-a]-pyrimidin und dessen Salze;

– 7-Methoxy-5-methyl-2-(5-methyl-1,2,4-oxadiazol-3-yl)-6-propylimidazo-[1,2-a]-pyrimidin und dessen Salze;

– 6-Ethyl-5-methyl-2-(5-methyl-1,2,4-oxadiazol-3-yl)-7-methylthioimidazo-[1,2-a]-pyrimidin und dessen Salze;

– 6,7,8,9-Tetrahydro-5-methoxy-2-(5-methyl-1,2,4-oxadiazol-3-yl)-imidazo-[1,2-a]-chinazolin und dessen Salze.

6. Verfahren zur Herstellung von Imidazo-[1,2-a]-pyrimidinen der Formel (I) gemäß Anspruch 1, worin $R_1$ für einen 1,2,4-Oxadiazol-5-yl-Rest steht, sowie von deren Salzen, dadurch gekennzeichnet, daß man ein Produkt der Formel (II)

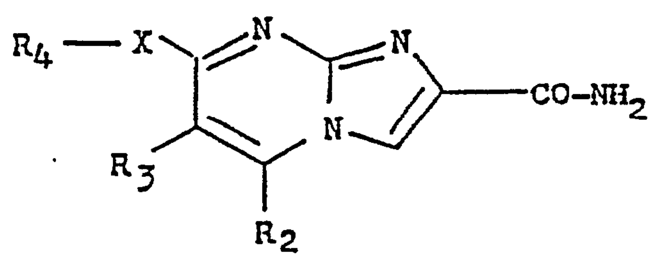

(II)

worin $R_2$, $R_3$, $R_4$ und X die angegebene Bedeutung besitzen, mit einem Produkt der Formel (III):

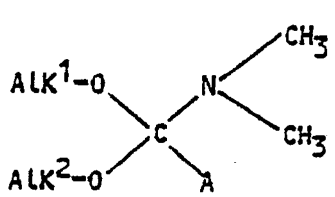

(III)

worin $Alk^1$ und $Alk^2$ jeweils für eine Alkylgruppe mit 1 bis 3 Kohlenstoffatomen stehen und A ein Wasserstoffatom, einen Alkylrest mit 1 bis 3 Kohlenstoffatomen oder einen Alkenylrest mit 2 bis 5 Kohlenstoffatomen bedeutet, umsetzt, um zu einem Produkt der Formel (IV):

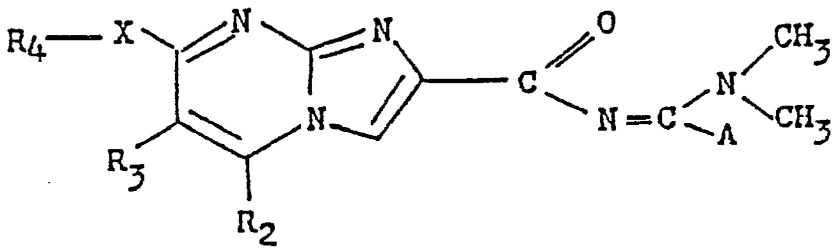

(IV)

zu gelangen, worin $R_2$, $R_3$, $R_4$, X und A die angegebene Bedeutung besitzen, hiernach dieses Letztgenannte mit Hydroxylaminhydrochlorid umsetzt, um zu dem gewünschten Produkt der Formel ($I_A$):

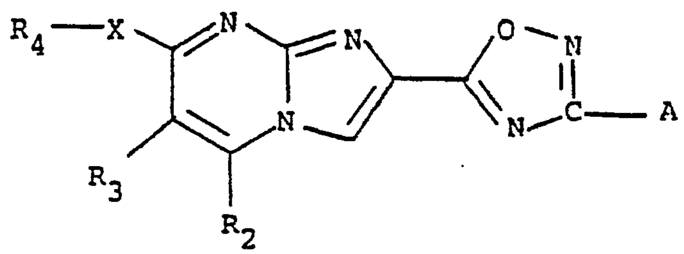

($I_A$)

zu gelangen, worin $R_2$, $R_3$, $R_4$, X und A die angegebene Bedeutung besitzen, und hiernach gegebenenfalls das so erhaltene Produkt der Formel (I) in ein Salz überführt.

7. Verfahren gemäß Anspruch 6, dadurch gekennzeichnet, daß:

a) die Umsetzung des Produkts der Formel (II) mit dem Produkt der Formel (III) durch Erhitzen des Reaktionsmilieus erfolgt;

b) die Umsetzung des Produkts der Formel (IV) mit dem Hydroxylaminhydrochlorid in Anwesenheit einer Base in einem organischen Lösungsmittel in Gegenwart eines Kolösungsmittels durchgeführt wird.

8. Verfahren zur Herstellung von Imidazo-[1,2-a]-pyrimidinen der Formel (I) gemäß Anspruch 1, worin $R_1$ einen 1,2,4-Oxadiazol-3-yl-Rest bedeutet, sowie von deren Salzen, dadurch gekennzeichnet, daß man ein Produkt der Formel (V):

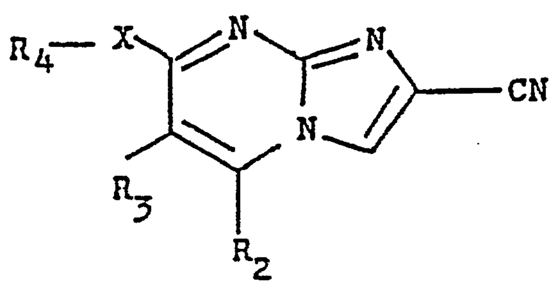

(V)

worin $R_2$, $R_3$, $R_4$ und X die angegebene Bedeutung besitzen, mit Hydroxylaminhydrochlorid umsetzt, um zu einem Produkt der Formel (VI):

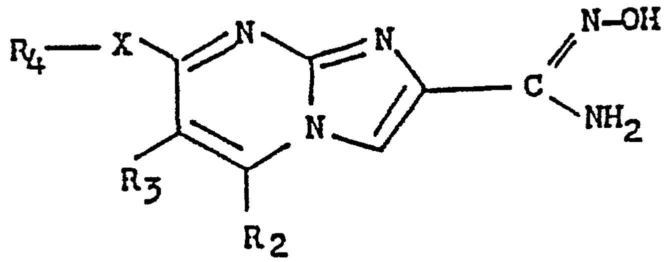

(VI)

zu gelangen, worin $R_2$, $R_3$, $R_4$ und X die angegebene Bedeutung besitzen, hiernach dieses Letztere mit einem funktionellen Säurederivat der Formel (IX):

D–COOH (IX)

worin D für ein Wasserstoffatom, einen Alkylrest mit 1 bis 3 Kohlenstoffatomen, der gegebenenfalls

durch ein oder mehrere Fluoratome substituiert ist, oder einen Alkenylrest mit 2 bis 5 Kohlenstoffatomen steht, umsetzt, um zu einem Produkt der Formel ($I_B$):

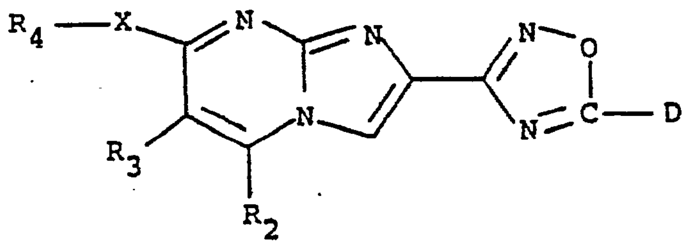

($I_B$)

zu gelangen, worin $R_2$, $R_3$, $R_4$, X und D die angegebene Bedeutung besitzen, und hiernach gegebenenfalls das so erhaltene Produkt der Formel (I) in ein Salz überführt.

9. Verfahren gemäß Anspruch 8, dadurch gekennzeichnet, daß:

a) die Umsetzung des Produkts der Formel (V) mit dem Hydroxylaminhydrochlorid durch Erhitzen unter Rückfluß des Reaktionsgemisches in einem organischen Lösungsmittel in Gegenwart einer Base erfolgt;

b) das funktionelle Säurederivat der Formel (IX)

D–COOH (IX)

ein Säureanhydrid oder ein Amidacetal der Formel (III')

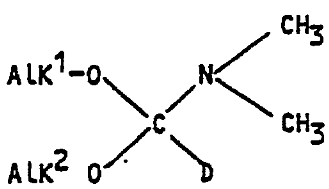

(III')

ist, worin $Alk^1$ und $Alk^2$ die vorstehenden Bedeutungen besitzen;

c) die Umsetzung des Produkts der Formel (VI) mit dem funktionellen Säurederivat D–COOH durch Erhitzen des Reaktionsmilieus erfolgt;

d) die Umsetzung des Produkts der Formel (VI) mit dem funktionellen Säurederivat D–COOH in Gegenwart einer Base in einem organischen Lösungsmittel unter Rückfluß erfolgt, wenn das reaktive Derivat ein starkes Dehydratationsmittel ist.

10. Verfahren zur Herstellung von Imidazo-[1,2-a]-pyrimidinen der Formel (I) gemäß Anspruch 1, worin $R_1$ einen 1,3,4-Oxadiazol-2-yl-Rest bedeutet, sowie von deren Salzen, dadurch gekennzeichnet, daß man ein Produkt der Formel (VII):

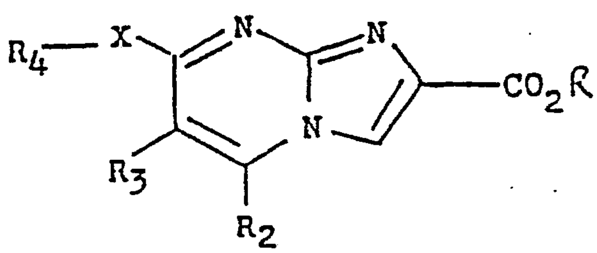

(VII)

worin $R_2$, $R_3$, $R_4$ und X die angegebene Bedeutung besitzen, und R den Rest einer Estergruppe wiedergibt, mit Hydrazinhydrat umsetzt, um zu einem Produkt der Formel (VIII):

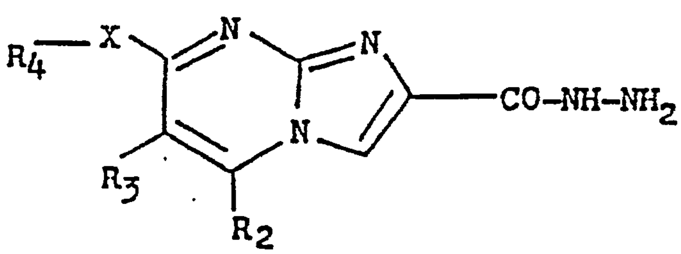

(VIII)

zu gelangen, worin $R_2$, $R_3$, $R_4$ und X die angegebene Bedeutung besitzen, hiernach dieses Letztere entweder mit einem Säureanhydrid oder einem Säurechlorid der Formel (IX):

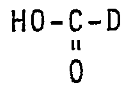

(IX)

worin D die angegebene Bedeutung besitzt, umsetzt, um zu einem Produkt der Formel (X):

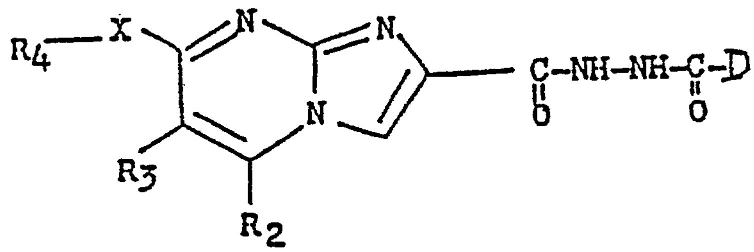

(X)

zu gelangen, oder mit einem Orthoester der Formel

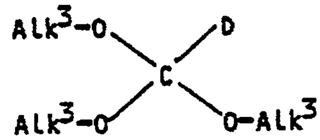

worin $Alk^3$ einen Alkylrest mit 1 bis 3 Kohlenstoffatomen bedeutet, umsetzt, um zu einem Produkt der Formel (XI):

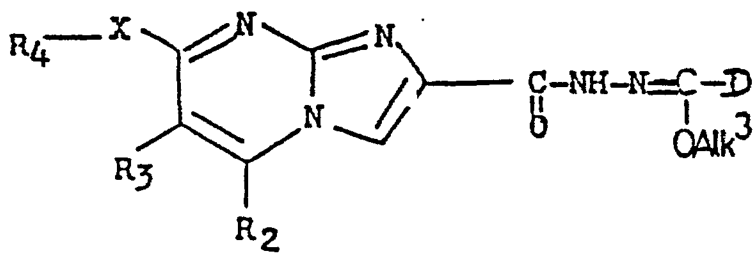

(XI)

worin $R_2$, $R_3$, $R_4$, X und D die angegebene Bedeutung besitzen und $Alk^3$ die vorstehende Bedeutung besitzt, zu gelangen und hiernach durch Erhitzen das Produkt der Formel (X) oder (XI) cyclisiert, um ein Produkt der Formel ($I_C$):

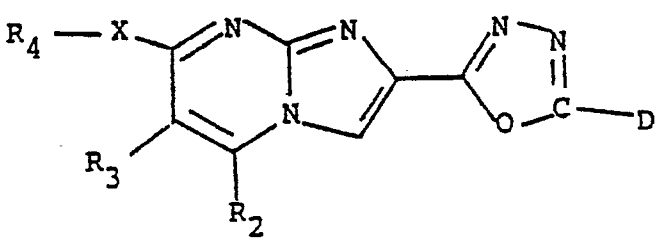

($I_C$)

worin $R_2$, $R_3$, $R_4$, X und D die angegebene Bedeutung besitzen, zu erhalten und danach gegebenenfalls das so erhaltene Produkt der Formel (I) in ein Salz überführt.

11. Verfahren gemäß Anspruch 10, dadurch gekennzeichnet, daß:

a) der Rest der Estergruppe, der durch R wiedergegeben wird, ein Alkylrest mit 1 bis 3 Kohlenstoffatomen ist;

b) die Umsetzung des Produkts der Formel (VII) mit Hydrazinhydrat durch Rückflußerhitzen des Reaktionsgemisches in einem organischen Lösungsmittel durchgeführt wird;

c) wenn das reaktive Derivat, das man mit dem Produkt der Formel (VIII) umsetzt, ein starkes Dehydratationsmittel ist, das Produkt der Formel (X) oder (XI) in situ in dem Reaktionsmilieu hergestellt wird

und die Cyclisierung in Gegenwart einer Base in dem Medium eines organischen Lösungsmittels bei einer Temperatur zwischen 25 und 45°C erfolgt;
d) wenn das reaktive Derivat, das man mit dem Produkt der Formel (VIII) umsetzt, kein starkes Dehydratationsmittel ist, das Produkt der Formel (X) oder (XI) isoliert wird und die Cyclisierungsreaktion durch Erhitzen des Reaktionsmilieus in Gegenwart von Phosphonylchlorid oder vorzugsweise in Gegenwart einer starken Säure durchgeführt wird.
12. Verfahren zur Herstellung von Imidazo-[1,2-a]-pyrimidinen der Formel (I) gemäß Anspruch 1, worin $R_1$ einen 1,3,4-Thiadiazol-2-yl-Rest bedeutet, sowie von deren Salzen, dadurch gekennzeichnet, daß man ein Produkt der Formel (VIII):

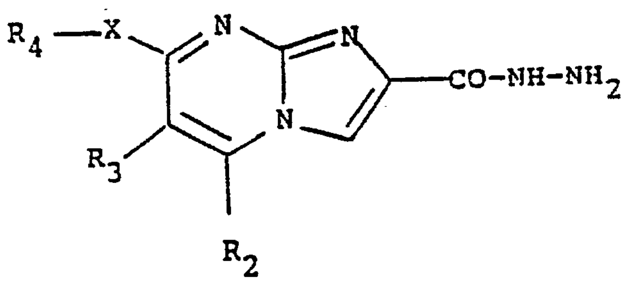

(VIII)

worin $R_2$, $R_3$, $R_4$ und X die angegebene Bedeutung besitzen, mit dem funktionellen Derivat einer Säure der Formel (IX')

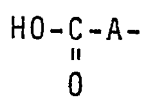

(IX')

worin A die angegebene Bedeutung besitzt, umsetzt, um zu einem Produkt der Formel (XII):

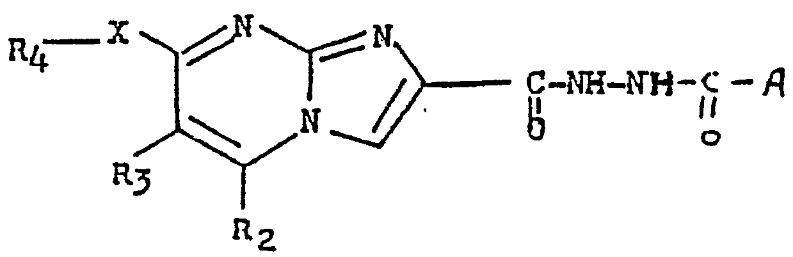

(XII)

worin $R_2$, $R_3$, $R_4$, X und A die angegebene Bedeutung besitzen, zu gelangen, hiernach dieses Letztgenannte mit dem Lawesson-Reagenz in dem Medium eines organischen Lösungsmittels umsetzt, um zu einem Produkt der Formel ($I_D$):

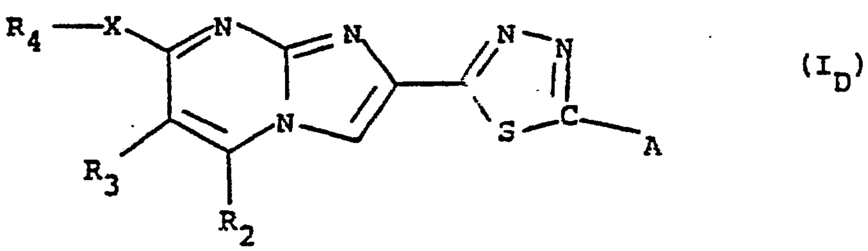

($I_D$)

worin $R_2$, $R_3$, $R_4$, X und A die angegebene Bedeutung besitzen, zu gelangen und hiernach gegebenenfalls das so erhaltene Produkt der Formel (I) in ein Salz überführt.
13. Verfahren gemäß Anspruch 12, dadurch gekennzeichnet, daß:
– die Umsetzung mit dem Lawesson-Reagenz oder (2,4-bis-4-methoxyphenyl)-1,3-dithia-2,4-diphosphetan-2,4-disulfid der Formel:

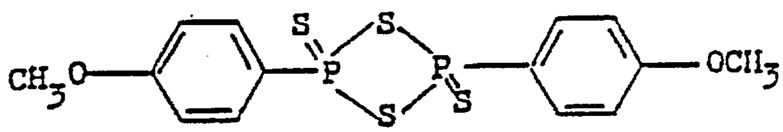

in dem Medium eines organischen Lösungsmittels unter Rückfluß des Reaktionsgemisches durchgeführt wird.

14. Arzneimittel, dadurch gekennzeichnet, daß sie aus den Imidazo-[1,2-a]-pyrimidinen der Formel (I) gemäß Anspruch 1 sowie aus deren pharmazeutisch verträglichen Salzen bestehen.

15. Arzneimittel, dadurch gekennzeichnet, daß sie aus den Imidazo-[1,2-a]-pyrimidinen gemäß einem der Ansprüche 2, 3 oder 4 sowie aus deren pharmazeutisch verträglichen Salzen bestehen.

16. Arzneimittel, dadurch gekennzeichnet, daß sie aus den Imidazo-[1,2-a]-pyrimidinen gemäß Anspruch 5 bestehen.

17. Pharmazeutische Zusammensetzungen, dadurch gekennzeichnet, daß sie als Wirkstoff zumindest eines der Arzneimittel gemäß einem der Ansprüche 14 bis 16 enthalten.